# EUROPEAN PATENT APPLICATION

(11) **EP 4 292 597 A1**
(43) Date of publication of application: **20.12.2023**
(21) Application number: 22179332.6
(22) Date of filing: 15.06.2022
(51) Int. Cl.: A61K 31/716, A61P 37/04

(54) **BETA-GLUCAN PARTICLE FOR TRAINED IMMUNITY**

(71) Applicant: The Provost, Fellows, Scholars and other Members of Board of Trinity College Dublin, Dublin 2 (IE); University College Dublin, Dublin 4 (IE)
(72) Inventor: Sheedy, Frederick J., Dublin 2 (IE); Charles-Messance, Hugo, Dublin 2 (IE); Hackett, Emer, Dublin 2 (IE); Mitchelson, Kathleen, Dublin 4 (IE); Roche, Helen, Dublin 4 (IE)
(74) Representative: Purdylucey Intellectual Property

(57) **Abstract**

An effective amount of a *Saccharomyces* cerevisiae-derived (1→3)-β-D-glucan particle for use in a method to program bone marrow hematopoietic stem and progenitor cells (HSPC) to promote multipotent progenitor (MPPs) expansion in favour of myelopoiesis in a subject.

## Description

### Field of the invention

The invention relates to a whole β-glucan particle for use in inducing trained immunity in a subject. More specifically, the invention relates to the use of a whole β-glucan particle for use in programming bone marrow hematopoietic stem & progenitor cell (HSPC) to provide multipotent progenitor (MPPs) expansion in favour of myelopoiesis.

### Background of the Invention

In vertebrates, the immune system's function relies on two main pillars to fight off infections and pathogens: the innate immune system and the adaptive immune system. The innate immune system is the first line of defence against pathogens, while the adaptive immune system builds up a coordinated and specific response. The adaptive immune system has been known for years to display immunological memory features. For instance, it is the adaptive immune system which is mostly solicited in the vaccination process.

Until 2011, it was believed that patrolling immune cells belonging to the innate immune system, such as monocytes and macrophages, lacked immune memory. Since this time however, several studies revealed that innate immune cells, exposed to pathogens and danger signals could display features of immunological memory later upon re-infection. This phenomenon has been termed 'trained immunity'. As, patrolling innate immune cells provide our body's first immune response against invading organisms, targeting innate immune cells and 'priming' them could greatly enhance the body's ability to improve immune function and resistance to infection.

Exposure to microbial stimuli has been shown to metabolically and epigenetically alter cells. Innate immune genes (inflammatory cytokines, chemokines) become epigenetically primed at the chromatin level, resulting in altered - often enhanced - responses with accelerated kinetics upon maturation and re-challenge (Netea, M.G. et al., Defining trained immunity and its role in health and disease. Nat Rev Immuno, 2020).

It has been shown previously that trained immunity can successfully be induced by prior exposure to β-glucan from the cell wall of pathogenic yeasts, including *Candida albicans*, and is mechanistically driven in blood-borne monocytes by long-term epigenetic and metabolic reprogramming. Recent discoveries established that trained immunity acts at the whole-organism level, targeting the bone marrow hematopoietic stem & progenitor cell (HSPC) compartment, thereby providing sustained protection against various pathogens.

Much of this work has been demonstrated using fungal β-glucan derived from *C*. *albicans* (Quintin, J., et al Candida albicans infection affords protection against reinfection via functional reprogramming of monocytes. Cell Host Microbe, 12 223-232, 2012; Cheng, S.C. et al. mTOR and HI-1 alpha-mediated aerobic glycolysis as metabolic basis for trained immunity. Science 345, (2014); Garcia-Valtanen, P., et al., Evaluation of trained immunity by beta-1, 3 (d)-glucan on murine monocytes in vitro and duration of response in vivo. Immunol Cell Biol 95, 601-610, (2017)). More recent work showing *in vivo* training through bone marrow HSPC reprogramming and myelopoiesis was demonstrated following intraperitoneal (IP) injection of macro-fungi (mushroom) *Trametes versicolor* or *C.albicans* β-glucan (Moorlag, S. et al. beta-Glucan Induces Protective Trained Immunity against Mycobacterium tuberculosis Infection: A Key Role for IL-1. Cell Rep 31, (2020); Mitroulis, I. et al. Modulation of Myelopoiesis Progenitors Is an Integral Component of Trained Immunity. Cell 172, 147-161 (2018).

The anti-tuberculosis vaccine, Bacillus Calmette-Guérin (BCG) has also been demonstrated to induce innate training, and mice injected intravenously or subcutaneously with BCG show an expansion of Lineage^{neg} c-Kit^{pos} Sca-1^{pos} (LKS+) cells numbers and proportion in the bone marrow, as well as an expansion of myeloid-biased progenitors, named MPP3, at the expense of lymphoid-biased progenitors, namely MPP4s (Kaufmann, E. et al. BCG Educates Hematopoietic Stem Cells to Generate Protective Innate Immunity against Tuberculosis. Cell 172, 176-190 (2018) (Kaufmann et al., 2018). Similarly, the intraperitoneal injection of a β-glucan peptide from *Trametes versicolor* was shown to induce a significant hematopoietic increase in LKS+ cells and MPP3s (Mitroulis et al., 2018).

β-glucans are a heterogeneous family of structural carbohydrate with multiple biological activities (Camilli, G., Tabouret, G. & Quintin, J. The Complexity of Fungal beta-Glucan in Health and Disease: Effects on the Mononuclear Phagocyte System. Front Immunol 9, 673, (2018)). More common yeast β-glucans, particularly food-grade baking, and brewer's yeast, contain larger molecular weight (MW) and more branched β-glucans to which various health benefits have been ascribed. Wellmune is a common and well-tolerated dietary food, beverage and supplement ingredient and is derived from the cell wall of baker's yeast (*Saccharomyces cerevisiae*). It is a whole glucan particle (WGP) with a backbone of glucose molecules linked via unique chemical links - beta 1,3 chains to which are attached glucose chains via beta 1,6 links. Clinical studies have demonstrated its ability to enhance immune function in humans, with protective effects against various respiratory tract infections.

So far, traditional strategies to improve immune function and resistance to infection have relied on vaccination, which targets the so-called adaptive immune system and promotes immune memory through the stimulation of disease-specific adaptive T-cells and B-cells. Therefore, showing how a common food supplement derived from yeast could increase our immunity to a variety of infectious agents, including bacteria and viruses, is of great interest.

But no such trained immunity analysis has been conducted with Wellmune prior to the current invention. In particular, oral administration of Wellmune, or any other β-glucan variant, to an animal model, in order to reconstitute innate immune memory features in mature innate immune cells, had never been performed and published before.

The current invention serves to solve the problems of the prior art.

### Summary of the Invention

The current inventors are the first to demonstrate that the β-glucan, in particular (1→3)-β-D glucan derived from baker's yeast (*Saccaromyces cerevisiae)* (herein referred to as "β-glucan of the invention"), induces alterations in bone marrow precursors, namely the bone marrow hematopoietic stem & progenitor cell (HSPC), providing expansion of the progenitor populations at the level of the bone marrow, as well as enhancing the function of mature immune cells derived from these progenitors, when it is administered orally via food supplementation. Alterations include a skewing in bone marrow progenitors in favour of myeloid precursors, namely MMP3. Myeloid cells include neutrophils, monocytes, macrophages among others, and together make up a critical arm of the immune system, responsible for innate defence.

Without being bound by theory, oral administration of the β-glucan of the invention is thought to induce immunometabolic alterations in bone marrow progenitors, increasing in turn the levels of inflammatory cytokine production. This proinflammatory microenvironment is thought to promote multipotent progenitors (MPPs) expansion in favour of myelopoiesis. These innate immune cells (e.g., monocytes or neutrophils) are trained or "primed" and differentiate into mature innate immune cells, such as macrophages, with trained immunity characteristics. Thus, establishing that administration can be used to promote long-term trained immunity via reprogramming of hematopoietic precursors.

The effect after oral administration is surprising as it would have been considered in the field that oral administration would not induce such changes. Indeed, one would expect intestinal assimilation mechanisms to degrade the active component of the β-glucan of the invention, induce major structural changes in the molecule, or simply break down the molecule to pieces. These results suggest an ability of WGP particle to interact with the mucosal immune system and drive central innate immune memory.

The inventors compared the β-glucan of the invention to other β-glucans to define the structural and signalling requirements for training in monocytes *in vitro* but also in mice in *vivo.*

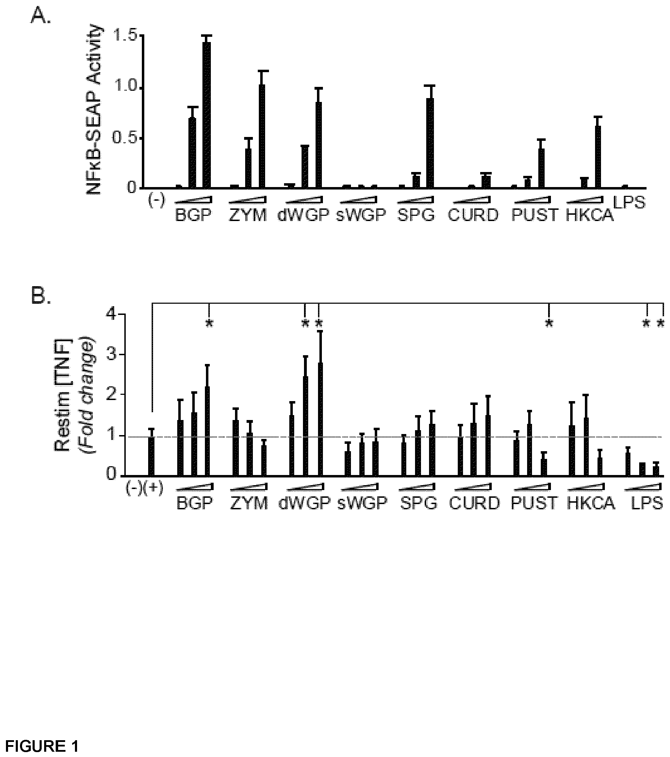

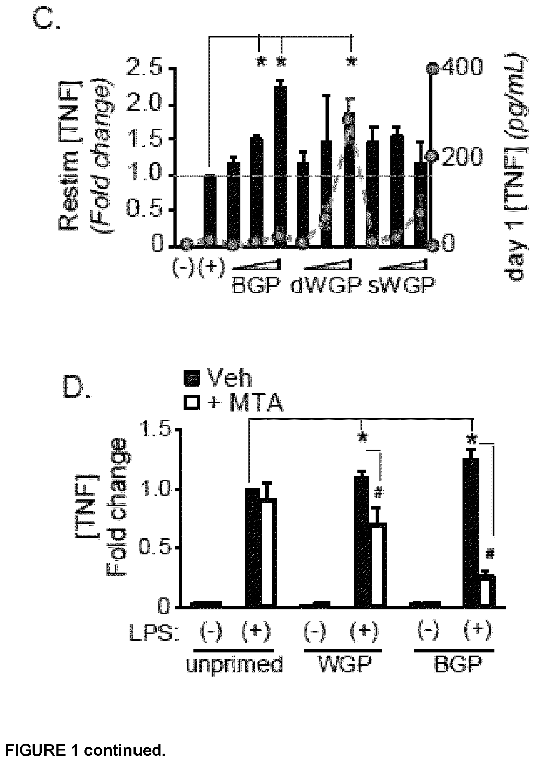

The inventors' work shows that the β-glucan of the invention can drive training effects in human innate immune cells, such as monocytes, in the lab as well as in injected mice at the bone-marrow level. The inventors have also shown that the β-glucan of the invention is more efficient than other β-glucan in inducing trained immunity features in human macrophages derived from trained monocytes *in vitro* (Figures 1 to 5). In addition, a soluble and non-particulate form of the β-glucan did not have the same effect.

The inventors have also shown that intraperitoneal (IP) injection the β-glucan of the invention in mice induces stronger changes in the bone marrow than other β-glucan variants. The inventors submit that oral administration of β-glucan of the invention induces a stronger hematopoietic skewing than other β-glucan variants.

The invention provides an effective amount of a *Saccharomyces cerevisiae-derived* (1→3)-β-D-glucan (herein "glucan of the invention") for use in a method to prime bone marrow hematopoietic stem and progenitor cells (HSPC) in a subject. The HSPC in said subject promote multipotent progenitor (MPPs) expansion in favour of myelopoiesis.

An aspect of the invention provides an effective amount of a *Saccharomyces cerevisiae-*derived (1→3)-β-D-glucan for use in a method to induce trained innate immunity in a subject. In this manner, the glucan is for use to mount an enhanced immune response against an invading pathogen. The glucan is a whole glucan particle.

An aspect of the invention provides an effective amount of a *Saccharomyces cerevisiae-*derived (1→3)-β-D-glucan for use in a method to produce trained innate immune cells in a subject. The trained innate immune cell may be a monocyte(s), and/or a mature innate immune cell, such as a macrophage. The glucan is a whole glucan particle.

In an embodiment of any aspect of the invention, the effective amount of a *Saccharomyces cerevisiae*-derived (1→3)-β-D-glucan of the invention may be formulated for oral administration or intraperitoneal injection (IP), preferably for oral administration.

Typically, the effective amount of the *Saccharomyces cerevisiae*-derived (1→3)-β-D-glucan is to prevent, delay the onset, or reduce the severity of a disease or infection in said subject.

Preferably, said *Saccharomyces cerevisiae*-derived (1→3)-β-D-glucan is administered in the early stages of a disease or infection or when a subject is suspected of having a disease or infection.

Preferably, the subject is one or more selected from the group comprising, an athlete, a subject experiencing or suffering from stress, an immunocompromised subject, a subject over 65 years of age, preferably over 75 years of age, a subject less than 16 years of age, a subject that has cancer or has recovered from cancer, and a subject with defective innate immunity.

In an embodiment, the subject is an athlete, typically a high-performance athlete. Such athletes have an increased susceptibility to upper respiratory tract infections at certain times, such as post marathon.

The subject may be a healthy subject. The subject may be of any age, preferably a subject over 65 years of age, preferably over 75 years of age, a subject less than 16 years of age.

In an embodiment, the glucan of the invention is a β-1,6 branched β-1,3 glucan (or "β-(1,3/1,6)) or a β-1,4 branched β-1,3 glucan particle.

Preferably, the (1→3)-β-D-glucan glucan is a β-1,6 branched β-1,3 glucan and has the following, typically repeating, structure:

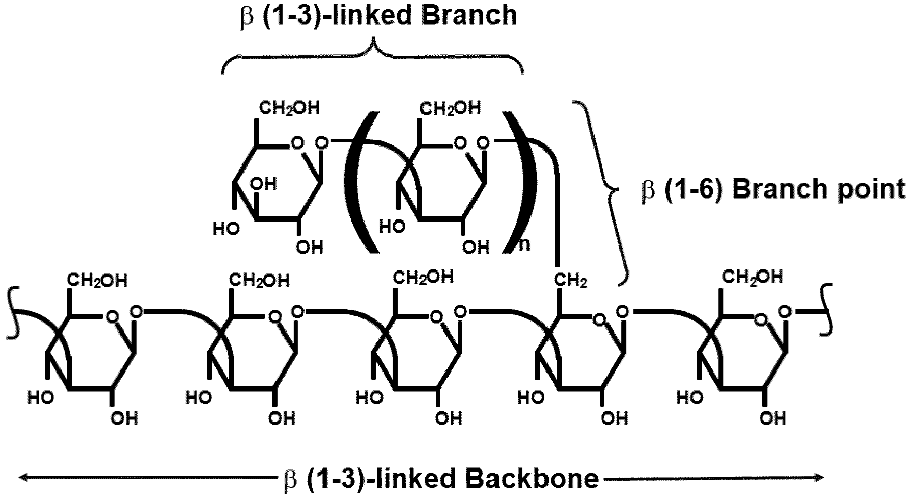

In an embodiment, the glucan of the invention consists essentially of β (1-6) an β (1-3) linked glucan.

In an embodiment, the 1,6 linked side chains of the glucan of the invention are in the range of 3-6 glucose molecules, e.g., 4-5 glucose molecules.

The glucan of the invention may have a degree of branching of from 3 to 5%, e.g., 4%.

In an embodiment, the glucan of the invention is a whole glucan particle (WGP).

In an embodiment, the WGP has a size of from about 1 to about 6 microns (or µm), or from about 2 to about 5 microns, or from about 3 to about 4 microns.

In an embodiment, the WGP is insoluble.

Typically, the glucan is one derived or obtained from the cell wall of baker's yeast.

In an embodiment, the glucan of the invention is provided as a dietary or food supplement comprising the glucan of the invention. It may be a food supplement enriched with the glucan of the invention. The supplement may be selected from the group comprising, but not limited to, tablets, capsules, gummies, and powders, beverages/drinks and energy bars.

In an embodiment, the food supplement comprises ≥ 75% beta 1,3/1,6 glucan on a dry weight basis.

In an embodiment, the supplement is Wellmune supplement.

The supplement comprises 80% or more beta-glucan.

Typically, the supplement comprises ≥ 75% beta 1,3/1,6 glucan on a dry weight basis, <3.5% protein, <10% fat, <3% ash, <8% moisture, <0.1mg/kg mercury, <0.5mg/kg lead, <1.0 mg/kg arsenic, and <1.0 mg/kg cadmium.

A method to program bone marrow hematopoietic stem and progenitor cells (HSPC) in a subject is provided, comprising administering the glucan of the invention to said subject. The HSPC in said subject promote multipotent progenitor (MPPs) expansion in favour of myelopoiesis.

A method to induce trained innate immunity in a subject is provided, the method comprising administering the glucan of the invention to said subject.

A method to produce trained innate immune cells in a subject is provided, the method comprising administering the glucan of the invention to said subject. The trained innate immune cell may be a monocyte(s), and/or a mature innate immune cell, such as a macrophage.

### Definitions and general preferences

All publications, patents, patent applications and other references mentioned herein are hereby incorporated by reference in their entireties for all purposes as if each individual publication, patent or patent application were specifically and individually indicated to be incorporated by reference and the content thereof recited in full.

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g., a feature, element, characteristic, property, method/process step or limitation) or group of integers (e.g., features, element, characteristics, properties, method/process steps or limitations) but not the exclusion of any other integer or group of integers. Thus, as used herein the term "comprising" is inclusive or open-ended and does not exclude additional, unrecited integers or method/process steps.

As used herein, the term "disease" or "condition" is used to define any abnormal condition that impairs physiological function and is associated with specific symptoms. The term is used broadly to encompass any disorder, illness, abnormality, pathology, sickness, condition, or syndrome in which physiological function is impaired irrespective of the nature of the aetiology (or indeed whether the aetiological basis for the disease is established). It therefore encompasses conditions arising from infection, trauma, injury, surgery, radiological ablation, poisoning or nutritional deficiencies.

As used herein, the term "treatment" or "treating" refer to an intervention (e.g., the administration of an agent to a subject) which cures, ameliorates or lessens the symptoms of a condition or disease or removes (or lessens the impact of) its cause(s). In this case, the term is used synonymously with the term "therapy". It can be manifested by a permanent or temporary improvement in the subject's condition. In this context it includes limiting and/or reversing disease progression.

As used herein the terms "prevention" or "preventing" refer to an intervention (e.g., the administration of an agent to a subject), which prevents or delays the onset or progression of a condition, or the severity of a condition in a subject, or reduces (or eradicates) its incidence within a treated population.

When used herein, the term "composition" should be understood to mean something made by the hand of man, and not including naturally occurring compositions. Compositions may be formulated in unit dosage form, i.e., in the form of discrete portions containing a unit dose, or a multiple or sub-unit of a unit dose.

The term "symptom" is defined as an indication of disease, illness, injury, or that something is not right in the body.

As used herein, the term "effective amount or a therapeutically effective amount" as applied to the glucan of the invention defines an amount that can be administered to a subject without excessive toxicity, irritation, allergic response, or other problem or complication, commensurate with a reasonable benefit/risk ratio, but one that is sufficient to provide the desired effect. The amount will vary from subject to subject, depending on the age and general condition of the individual, mode of administration and other factors. Thus, while it is not possible to specify an exact effective amount, those skilled in the art will be able to determine an appropriate "effective" amount in any individual case using routine experimentation and background general knowledge. A therapeutic result need not be a complete cure. A therapeutic result may be a permanent or temporary improvement in the subject's condition.

The term "subject" means a human or animal, more typically a mammal. In one aspect, the subject is a human.

"B-glucans" are polysaccharides found inside the cell wall of bacteria and fungus. They are glucose (D-glucose) polymers linked together by a 1→3 liner β-glycosidic chain core and differ from each other by their length and branching structures. The branches derived from the glycosidic chain core are variable and the two main groups of branching are 1→4 or 1 →6 glycosidic chains.

When used herein the term "(1→3)-β-D-glucan" is a glucan comprising D-glucose units with β-1,3 links.

"β-1,6 branched β-1,3 glucan (or "β-(1,3/1,6)) is composed of a backbone of glucose molecules linked via unique chemical links β1,3 chains to which are attached to glucose chains via β 1,6 links.

When used herein the term "trained immunity" refers the immune memory characteristics of innate immune cells mediated by epigenetic and/or metabolic reprogramming. Trained immunity provides protection against infections and an enhanced immune response on infection, typically in the absence of antibodies.

When used herein the term "immune memory" or "immunological memory" refers to the ability of the immune system to quickly and specifically recognize a pathogenic component that it recognizes as non-self and initiate an immune response based upon its past exposures. This can be sub-divided into traditional adaptive immune memory which relies on the recognition of specific antigens to drive antibodies and cellular responses through lymphocytes, or innate immune memory/trained immunity, in which certain stimuli like beta-glucans prime innate immune cells to later respond differently to a broad range of pathogenic components.

The "innate immune response" is the first line of defence against invading pathogens, activating as a physical and/or a chemical barrier to infectious agents. The innate immune cells include natural killer cells, macrophages, monocytes, neutrophils, dendritic cells, mast cells, basophils and eosinophils. The innate immune system functions to activate the adaptive immune system via antigen presentation.

When used herein the term "bone marrow hematopoietic stem and progenitor cells (HSPC)" refers to a population of precursor cells that possess the capacity for self-renewal and give rise to multilineage differentiation. Functionally distinct hematopoietic precursor subsets include multipotent progenitor 2-4 (MPP2, MPP3 and MPP4). MMP3 are a myeloid-cell associated lineage. MMP4 are lymphoid-associated. In the bone marrow (BM), HSPCs ensure blood cell homeostasis.

When used herein the term "trained monocyte" refers to a monocyte that has immune memory mediated by epigenetic and/or metabolic reprogramming. For instance, in this context "trained monocyte" is one trained by exposure to beta-glucan in the lab, or a monocyte derived from the bone-marrow of an animal/subject who was exposed to beta-glucan. Trained monocytes typically exhibit enhanced pro-inflammatory cytokine responses to restimulation with TLR ligands (Saeed et al., Epigenetic programming of monocyte-to-macrophage differentiation and trained innate immunity, Science 2014).

When used herein the term "trained macrophage" refers to a macrophage (sentinel cell of the innate immune system) derived from the bone-marrow (via monocytes) or an animal/subject exposed to beta glucan/Wellmune or other driver of trained immunity, with an enhanced function, mediated by epigenetic and/or metabolic reprogramming.

When used herein the term "haematopoiesis" refers to the formation of blood cellular components. All cellular blood components are derived from hematopoietic stem cells (HSCs). HSCs are the stem cells that give rise to bother blood cells. In human adults, haematopoiesis occurs in the red bone marrow in the core of most bones.

When used herein the term "myeloid cell" refers to blood cells that arise from a progenitor cell for granulocytes, monocytes, erythrocytes, or platelets. This progenitor cell may be a myeloid progenitor or precursor cell, such as a common myeloid progenitor (CMP). It may be specifically from the lineage of the myeloblast, i.e., myelocytes and monocytes.

When used herein the term "myelopoiesis" refers to the process in which mature innate immune cells develop from a myeloid progenitor cell.

When used herein the term "multipotent progenitor (MPPs) expansion" refers to an increase in the relative proportion and/or absolute number of the MPP pool of cells in an animal/subject, typically in response to treatment.

When used herein the term "whole glucan particle" refers to a β-glucan isolated from glucan containing cell walls and substantially retaining the *in vivo* glucan morphology. WGP is not soluble and is preferably spherical. The WGP of the invention are typically pure glucan particles, e.g., 80%, or more, 90% or more, 95%, 98%, or 100% beta glucan. In an embodiment, the WGP exhibit a high-water holding capacity, as exhibited by their viscosity in aqueous solutions. For example, a WGP that is approximately 2 to 4 microns containing 5.5grams of glucan per decilitre has a viscosity of about 1000 centipoise. Methods to determine viscosity are known in the art, e.g. (US4992540).

Methods of preparing a WGP are known in the art. Exemplary methods are disclosed in the publications disclosing Wellmune^{®} herein. In an embodiment, the WGP is one produced by one or more of these methods. Generally, the method comprises extracting alkali soluble components from glucan containing cell wall without a prior description of said cell wall to produce whole glucan particles retaining the *in vivo* glucan morphology.

For example, *Saccharomyces cerevisiae* is selectively grown to obtain a pure culture and expanded in stainless steel fermentation vessels. Following fermentation, the cells are lysed by holding them at 45-55°C for approximately 24 hours. After autolysis the cell wall is separated from soluble yeast extract using a continuous centrifugal separator. The collected yeast cell wall is further processed through a series of alkali and hot water washes (70-90°C) to remove cell wall mannosylated proteins and any residual cellular lipids. In a subsequent acidification step the cell wall chitin is removed and the remaining purified beta-1,3/1,6 glucan slurry is washed in hot water, concentrated and pH adjusted as required. The resulting product is flash pasteurized and spray dried. This method results in a glucan that has retained its yeast cell like macro structure.

Another method comprises a culturing a *Saccharomyces Cerevisiae* yeast strain in a culture medium, harvesting whole yeast cells from said culture medium, contacting said whole yeast cells with an aqueous hydroxide solution at a pH of from about 4.0 to about 12.5 or a normality of from about 0.75 to about 1.5 and a temperature of from about 25 °C. to about 100 °C for a sufficient time to extract protein from said whole yeast cells to form aqueous insoluble whole glucan particles containing less than 1%, by weight, protein, said glucan particles substantially retaining the *in vivo* glucan three-dimensional structure and typically, consisting essentially of glucans having β (1-6) and β(1-3) linkages (US4810646).

It is not a linear soluble beta glucan (small molecular weight, e.g., 25 KDa or less) such as those disclosed by the prior art. In other words, it is intact.

### Brief Description of the Figures

The invention will be described with reference to the following Figures in which;
**Figure 1**. Dectin-1 activating β-glucans differentially drive monocyte training. **A)** hDectin1-HEK293 NFκB-SEAP reporter cells were incubated with varying concentrations of the following β-glucans (BGP, Zymosan (ZYM), Wellmune^{®} dispersible (dWGP), Wellmune^{®} soluble β-glucan derived from heat-treated dWGP (sWGP), Schizopylan (SPG), Curdlan (CURD) and Pustulan (PUST) all at 1,10 & 100 µg/mL β-glucan, heat-killed *Candida albicans* (HKCA) at 10⁴, 10⁵ & 10⁶ cells/mL, or LPS (10 ng/mL). SEAP activity in supernatants was measured 6h post-stimulation and quantified relative to untreated cells (-). **B)** Human monocytes were exposed to the indicated β-glucans as above, or LPS (1, 10 & 100 ng/mL) 24h-post isolation, washed & allowed to mature to monocyte-derived macrophages in the presence of MCSF. 5-days post isolation cells were restimulated with LPS (10 ng/mL, 6h) & TNF production measured relative to untrained cells (-/+). **C)** hMDM were trained & restimulated as above using BGP, dWGP & sWGP and TNF production upon LPS restimulation (10 ng/mL, 6h - black bars, left axis) compared to TNF produced by monocytes 24h post-Training (blue line, right axis). **D)** Human monocytes were exposed to 5'methylthioadenosine (MTA, 1 mM) 1h prior to Training with BGP or dWGP for 24h, washed, matured and restimulated with LPS (10 ng/mL, 6h) as before. Data is mean SEAP quantification ± sem for n=3 independent experiments (A), mean TNF quantification ± sem for n=4 independent donors (B), mean TNF fold-change over trained cell ± sem for n=3 (C) or n=6 (D) independent experiments. * indicates P < 0.05 for post-hoc t-tests for indicated comparisons between groups.

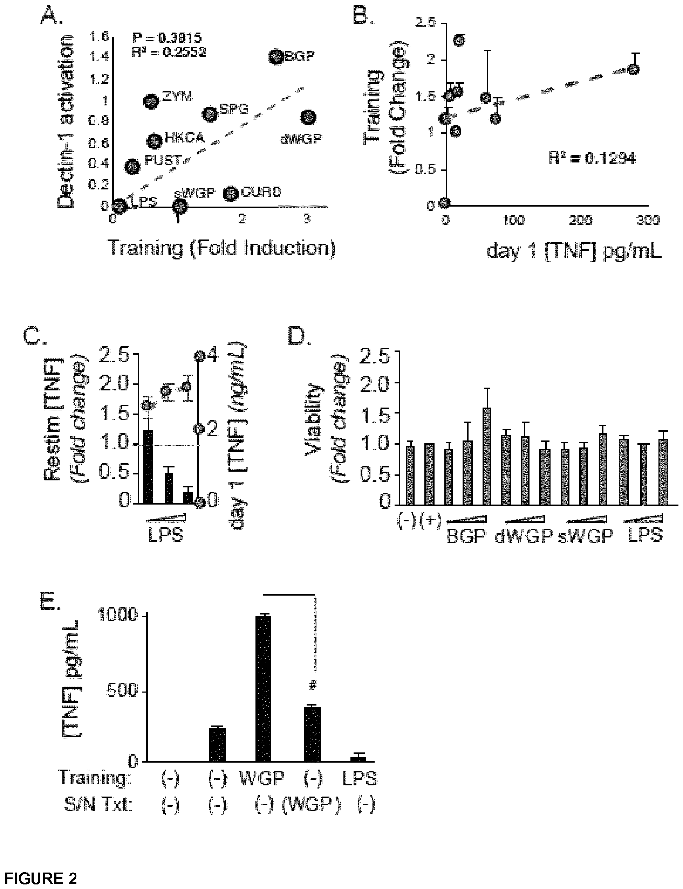

**Figure 2**. Early pro-inflammatory signaling and viability in β-glucan treated monocytes. **A-C)** Correlation analysis between relative changes in TNF production after LPS restimulation (training) and NFκB-linked SEAP production in hDectin1-HEK293 reporter cells (Dectin-1 activation, A) or TNF production 24h post treatment with the indicated β-glucans (day 1 TNF, B); (BGP, Zymosan (ZYM), Wellmune^{®} dispersible (dWGP), Wellmune^{®} soluble (sWGP), Schizopylan (SPG), Curdlan (CURD) and Pustulan (Pust) all at 100 ug/mL β-glucan, heat-killed Candida albicans (HKCA) at 10⁴ cells/mL, or overlay on graph above training for LPS (10 ng/mL, C). **D)** Viable hMDM 5-days post training with the indicated training agents (BGP, dWGP & sWGP 1, 10 & 100 µg/mL or LPS 1, 10, 100 ng/mL) measured by Crystal Violet Blue staining and expressed relative to untrained hMDMs. **E)** Supernatants from dWGP-trained monocytes (10 µg/mL) were transferred to untrained monocytes (S/N Txt), left for 5-days and both WGP-trained and S/N Txt hMDM were restimulated with LPS (10 ng/mL, 6h) and TNF production measured. A) plots mean data from Fig. 1A (n=4-6 independent donors per condition), B plots mean data from cells as treated in Fig. 1C (n=3 independent experiments), C plots both mean TNF fold-change over trained cell (left axis) or mean TNF value (right axis) ± sem for n=3 experiments. D is mean OD relative to untrained cells for n=3 experiments. E plots mean TNF value ± sem for n=3 experiments. # indicates P < 0.05 for post-hoc t-tests for indicated comparisons between groups.

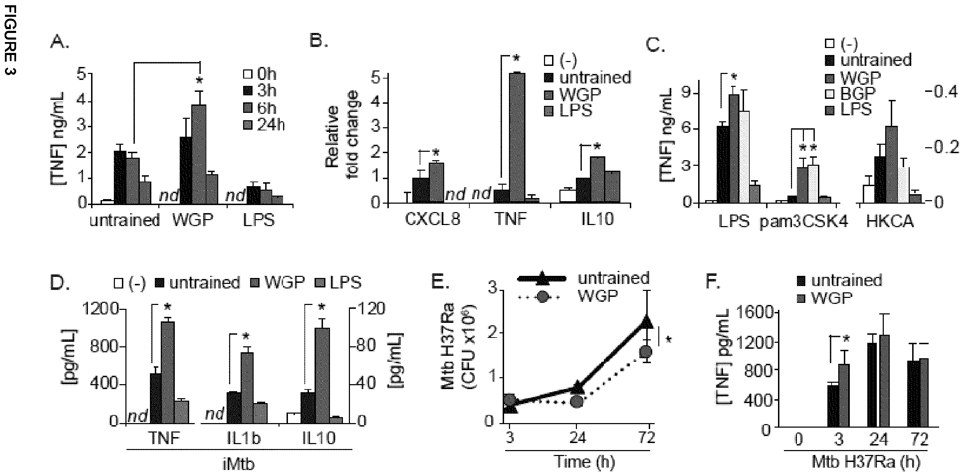

**Figure 3****.** Macrophage responses to restimulation in β-glucan trained macrophages. **A)** Monocytes trained with Wellmune^{®} dispersible (dWGP, 10 µg/mL, blue bars) or tolerized with LPS (10 ng/mL, gray bars) were matured to hMDM and restimulated with LPS (10 ng/mL) for the indicated times (0-24h) and TNF production measured. **B)** hMDM derived from trained monocytes as above were restimulated with LPS (10 ng/mL) and CXCL8 at 3h, TNF at 6h and IL-10 production measured 24h post-stimulation and expressed relative to untrained cells. **C)** hMDM derived from trained monocytes as above were restimulated with LPS (100 ng/mL), pam3CSK4 (100 µg/mL) or HKCA (10⁶cells/mL) and TNF production measured after 6h. **D)** hMDM derived from dWGP-trained (10 µg/mL, blue bars) or LPS-tolerised (10 ng/mL, grey bars) monocytes were treated with irradiated Mycobacterium tuberculosis H37Rv (iMtb, 500 ug/mL) for 24h and production of the indicated cytokines measured. **E-F)** hMDM derived from dWGP-trained monocytes (10 µg/mL, blue bars) were infected with Mtb H37Ra (MOI 5 bacteria/cell) and intracellular bacterial survival measured by CFU analysis at the indicated times post-infection (E) or TNF production measured in culture supernatants (F). All data is mean absolute values (A, C-F) or fold-change in TNF over untrained (B) ± sem for n=3 (A-B, D), n=4 (C) and n=5 (E-F) independent experiments. * indicates P < 0.05 for post-hoc t-tests for indicated comparisons between groups.

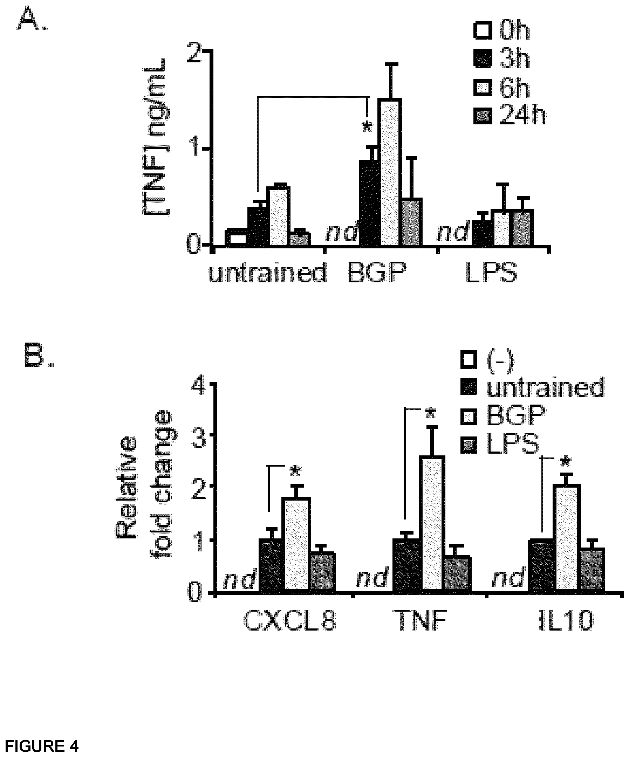

**Figure 4****.** BGP-induced trained responses in human monocyte-derived macrophages. **A)** Monocytes trained with BGP (10 µg/mL, yellow bars) or tolerized with LPS (10 ng/mL, gray bars) were matured to hMDM and restimulated with LPS (10 ng/mL) for the indicated times (0-24h) and TNF production measured. **B)** hMDM derived from trained monocytes as above were restimulated with LPS (10ng/mL) and CXCL8 at 3h, TNF at 6h and IL-10 production measured 24h post-stimulation and expressed relative to untrained cells. Data is mean absolute values (A) or fold-change in TNF over untrained (B) ± sem for n=3 independent experiments. * indicates P < 0.05 for post-hoc t-tests for indicated comparisons between groups.

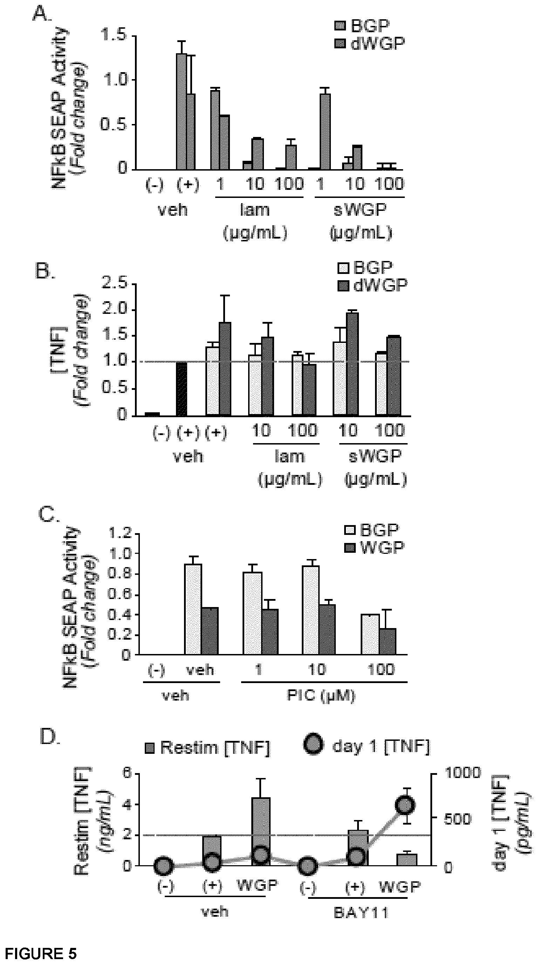

**Figure 5****.** Dectin-1/Syk/NFκB signalling is required for Wellmune^{®} dispersible (dWGP)-induced monocyte training. **A)** hDectin1-HEK293 NFκB-SEAP reporter cells were incubated with laminarin or Wellmune^{®} soluble (sWGP) at the indicated concentrations for 1h prior to treatment with BGP (blue) or dWGP (gray) at 100 µg/mL. SEAP activity in supernatants was measured 6h post-stimulation and quantified relative to untreated cells (-). **B)** Monocytes were incubated with laminarin or sWGP at the indicated concentrations for 1h prior to training with BGP (yellow) or dWGP (blue) at 100 µg/mL for 24h. Mature macrophages were then restimulated with LPS (10 ng/mL, 6h) and TNF production measured. **C)** hDectin1-HEK293 NFκB-SEAP reporter cells were incubated with the indicated concentrations of picetannol (PIC) for 1h prior to treatment with BGP (yellow) or dWGP (blue) at 100 µg/mL. SEAP activity in supernatants was measured 6h post-stimulation and quantified relative to untreated cells (-). **D)** Monocytes were incubated with BAY-11087 (5 µM, BAY) prior to training with dWGP (100 µg/mL, blue) and TNF production measured after 24h and compared to fold-change in TNF production in LPS restimulated mature macrophages. Data in A & C) is mean value ± sem for n=3 (A) and n=4 (C) independent experiments. Data in B is mean fold change in TNF relative to untrained cells for n=2 independent experiments. Data in D is mean TNF value ± sem for n=5 independent experiments.

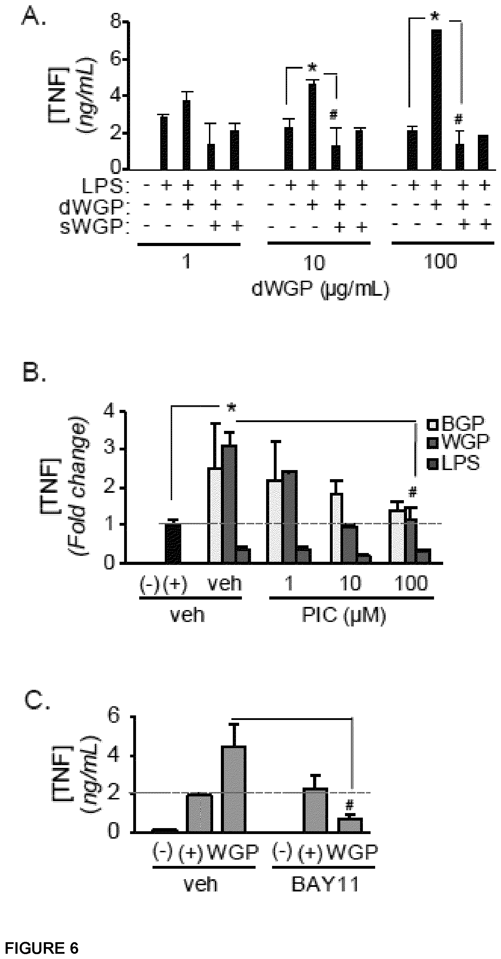

**Figure 6****.** Canonical Dectin-1 signaling is required for Wellmune^{®} dispersible (dWGP)-induced monocyte training. **A)** Monocytes were incubated with Wellmune^{®} soluble (sWGP, 100 µg/mL) for 1h prior to training with dWGP (1,10 & 100 ug/mL) for 24h. Mature macrophages were then restimulated with LPS (10 ng/mL, 6h) and TNF production measured. **B-C)** Prior to training with BGP (yellow), dWGP (blue) or tolerized with LPS (gray), monocytes were incubated with the indicated concentrations of picetannol (PIC, between 4, 10 and 30 µM, B) or BAY-11087 (BAY11, 5 µM, C). Mature macrophages were then restimulated with LPS (10 ng/mL, 6h) and TNF production measured. Data in A & C is mean TNF value ± sem for n=3 (A) and n=5 (C) independent experiments. Data in B is mean fold change in TNF relative to untrained cells for n=3 independent experiments. * indicates P < 0.05 for post-hoc t-tests for comparisons between untrained cells and # indicates P < 0.05 for comparisons between no inhibitor cells.

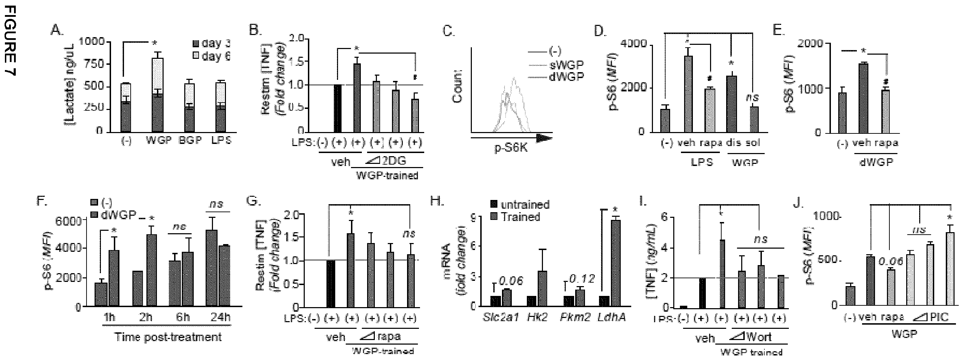

**Figure 7****.** Wellmune^{®} dispersible (dWGP) drives mTOR-mediated glycolysis for training. **A)** Monocytes were trained with dWGP or BGP (10 µg/mL) or tolerized with LPS (100 ng/mL) for 24h, media removed and allowed to mature. Supernatants were tested for extracellular lactate production at the indicated days post-training. **B)** Prior to training with dWGP, monocytes were incubated with the 2 deoxyglucose (2DG, 1, 5 & 10 µM). Mature macrophages were then restimulated with LPS (10 ng/mL, 6h) and TNF production measured. **C)** Representative histogram plot of monocytes treated with sWGP or dWGP (10 µg/mL, 2h) or left untreated and subjected to intracellular staining for p-S6K detected by flow cytometry. **D-E)** Monocytes were pre-treated with rapamycin (rapa, 10 nM) for 15 min prior to stimulation with LPS (10 ng/mL) or dWGP or sWGP (both at 10 µg/mL) for 2h. p-S6K activity was measured by flow cytometry. **F)** Monocytes were stimulated with dWGP (10 µg/mL) for the indicated times and p-S6K activity measured by flow cytometry. **G)** Prior to training with dWGP, monocytes were incubated with rapamycin (rapa, between 1, 10 and 100 nM). Mature macrophages were then restimulated with LPS (10 ng/mL, 6h) and TNF production measured. **H)** Monocytes were trained with β-glucan for 24h and allowed to mature to macrophages for 5-days. Expression of the indicated genes was measured by qPCR and expressed relative to untrained cells. **I)** Prior to training with dWGP, monocytes were incubated with the wortmannin (Wort, between 0.1, 1 and 10 µM). Mature macrophages were then restimulated with LPS (10 ng/mL, 6h) and TNF production measured. **J)** Monocytes were pre-treated with picetannol (PIC, between 4, 10 and 30 µM) for 15 min prior to stimulation with dWGP (10 µg/mL, 2h). p-S6K activity was measured by flow cytometry. Data in A) is mean Lactate value ± sem for n=4 independent experiments. Data in B & G is mean fold change in TNF relative to untrained cells ± sem for n=7 (B) and n=6 (G) independent experiments. Flow cytometry data ((D-F & J) is mean MFI ± sem for n=3 independent experiments. Data in H is mean fold change in trained cells relative to untrained for the indicated genes ± sem for n=3 independent experiments. Data in I is mean TNF values ± sem for n=3 independent experiments. * indicates P < 0.05 for post-hoc t-tests for comparisons between untrained cells, # indicates P < 0.05 for comparisons between no inhibitor cells and n.s. indicates a non-significant change.

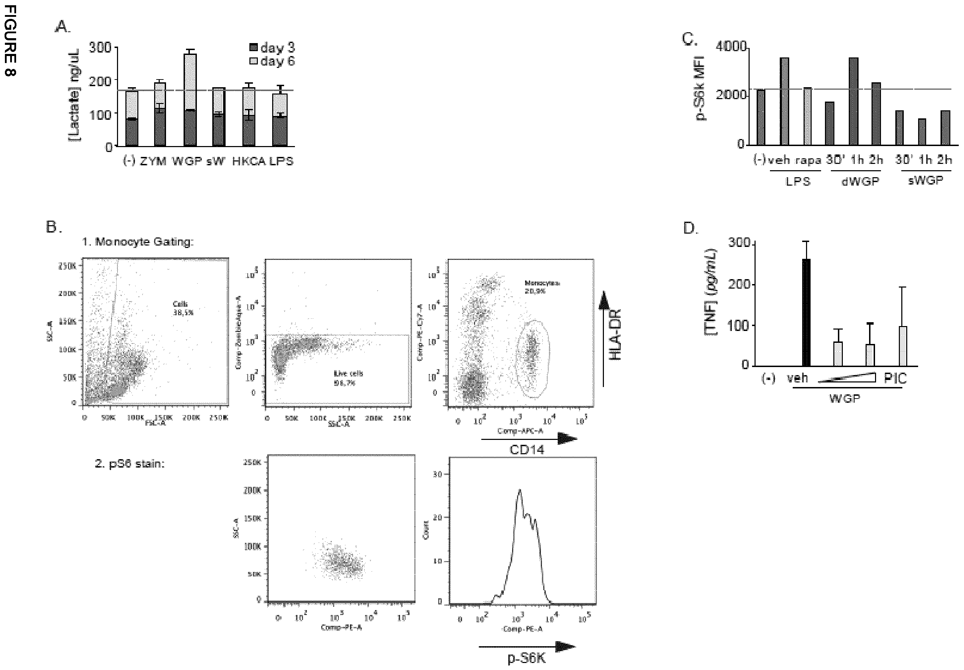

**Figure 8****.** Metabolic analysis in β-glucan-trained macrophages. **A)** Lactate production in monocytes treated with Zymosan (ZYM), Wellmune^{®} dispersible (dWGP), Wellmune^{®} soluble (sWGP) all at 10 µg/mL, heat-killed Candida albicans (HKCA, 10⁴ cell/mL) or LPS (100 ng/mL) for 24h and supernatants sample at the indicated days post-training. **B)** Gating strategy used for intracellular staining of monocytes for p-S6-kinase activity. **C)** Monocytes were stimulated with dWGP or sWGP (100 µg/mL) for the indicated times or treated with LPS (100 ng/mL) in the presence or absence of rapamycin (rapa, 10 nM) and p-S6K activity measured by flow cytometry. **D)** Monocytes were pre-treated with picetannol (PIC, between 4, 10 and 30 µM) for 15 min prior to stimulation with LPS (100 ng/mL) or dWGP (10 µg/mL). TNF production was measured in supernatants after 24h. Data in A) is mean Lactate value ± sd for n=2 independent experiments and D) is mean TNF value ± sem for n=3 independent experiments.

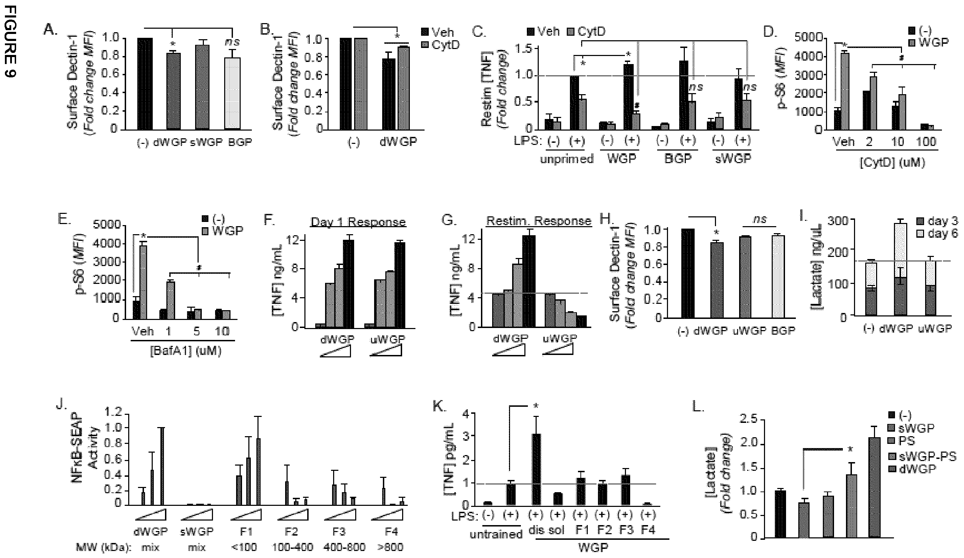

**Figure 9****.** Phagocytosis of intact Wellmune^{®} dispersible (dWGP) drives mTOR-mediated glycolysis. **A-B)** Monocytes were treated with the indicated β-glucan (10 ug/mL) for 15 min (A) or pre-treated with cytochalasin D (CytD, 10 µM) for 1h prior to pre-treatment with dWGP (100 ug/mL) for 15 min (B). Cells were immediately fixed and stained for surface Dectin-1 expression measured by flow cytometry. **C)** Monocytes were incubated with cytochalasin D (CytD, 10 µM) and subsequently trained with the indicated β-glucan (10 ug/mL) for 24h. Mature macrophages were then restimulated with LPS (10 ng/mL, 6h) and TNF production measured. **D-E)** Monocytes were pre-treated with cytochalasin D (CytD, between 1, 10 and 100 µM) or bafilomycin A1 (bafA1, between 1, 5 and 10 µM) for 15 min prior to stimulation with dWGP (10 µg/mL) for 2h. p-S6K activity was measured by flow cytometry. **F-G)** Monocytes were trained with dWGP or unsonicated WGP (uWGP) at 10 (orange), 50 (grey) or 100 µg/mL (black bars) for 24h or left untreated (blue bars) and TNF production in supernatants measured (F). Mature macrophages were restimulated with LPS (10 ng/mL, 6h) and TNF production measured (G). **H)** Surface Dectin-1 staining in monocytes incubated with the indicated β-glucan (10 µg/mL) for 15 min measured by flow cytometry. **I)** Extracellular lactate production in monocytes trained with dWGP or uWGP (100 µg/mL) for 24h and allowed to mature and sampled at the indicated times post-training. **J)** hDectin1-HEK293 NFκB-SEAP reporter cells were incubated with dWGP, sWGP or defined fractions of sWGP F1-F4 (MW ranges indicated below, kDa) at 1, 10 and 100 µg/mL. SEAP activity in supernatants was measured 6h post-stimulation and quantified relative to untreated cells (-). **K)** Monocytes trained with dWGP (dis), Wellmune^{®} soluble (sWGP, sol) or the indicated soluble fractions F1-F4 (10 µg/mL) for 24h. Mature macrophages were then restimulated with LPS (10 ng/mL, 6h) and TNF production measured. **L)** Monocytes were stimulated with 200 µg/mL aminated polystyrene particles (AM-PS) alone or conjugated with sWGP (sWGP-PS), the equivalent amount of sWGP roughly corresponding to 200 µg/ml of sWGP-PS (1 µg/ml), dWGP (100 µg/ml) or left untreated for 3 days and extracellular Lactate production measured. Data in A-B, D-E & H are mean MFI ± sem for n=3 independent experiments. Data in B is mean fold change in TNF relative to untrained cells ± sem for n=7 independent experiments. Data in F-G & K is mean TNF value ± sem for n=3 independent experiments. Data in I & L is mean Lactate value ± sem for n=3 independent experiments. Data in J is mean value ± sem for n=5 independent experiments. * indicates P < 0.05 for post-hoc t-tests for comparisons between untrained cells, # indicates P < 0.05 for comparisons between no inhibitor cells and n.s. indicates a non-significant change.

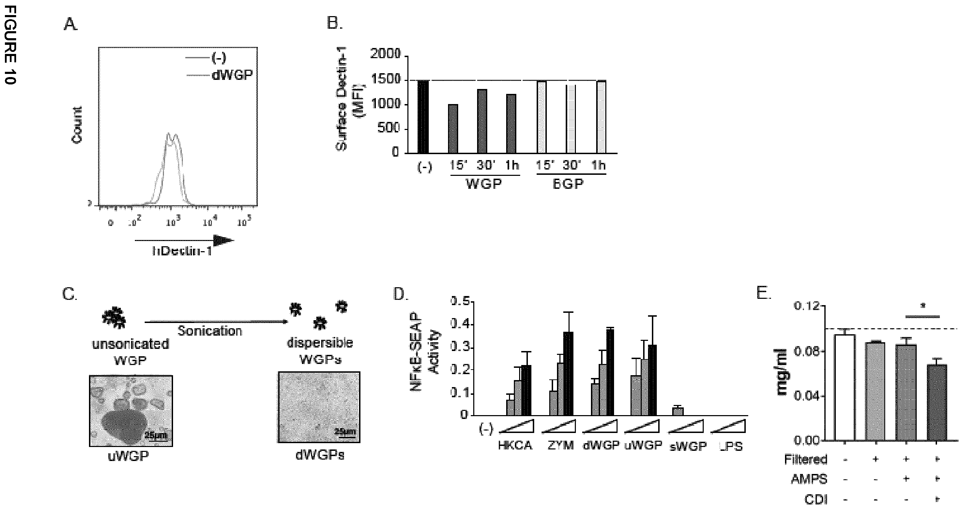

**Figure 10****.** Phagocytosis of particulate β-glucans. **A)** Representative histogram plot of monocytes treated with Wellmune^{®} dispersible (dWGP, 10 µg/mL, 2h) or left untreated and subjected to staining for surface Dectin-1 expression detected by flow cytometry. **B)** Monocytes were stimulated with dWGP or BGP (100 µg/mL) for the indicated times and surface Dectin-1 expression detected by flow cytometry. **C)** Schematic illustrating protocol to prepare single particle "dispersible" Wellmune^{®} (dWGP) from aggregated "unsonicated" Wellmune^{®} dispersible (uWGP) via sonication. Light micrographs represent monocyte cultures with each preparation. **D)** hDectin1-HEK293 NFκB-SEAP reporter cells were incubated with heat-killed Candida albicans (HKCA) at 10⁴, 10⁵ & 10⁶ cells/mL, Zymosan (ZYM), dWGP, uWGP or Wellmune^{®} soluble (sWGP) all at 1,10 & 100 µg/mL, or LPS (1,10 and 100 ng/mL). SEAP activity in supernatants was measured 6h post-stimulation and quantified relative to untreated cells (-). **E)** Sugar content of sWGP preparations after filtration following incubated with 2 µg aminated polystyrene particles (AM-PS) in the presence or absence of conjugating agent CDI. Data in D is mean value ± sem for n=2 independent experiments. Data in E is mean ± sd for triplicate determinations.

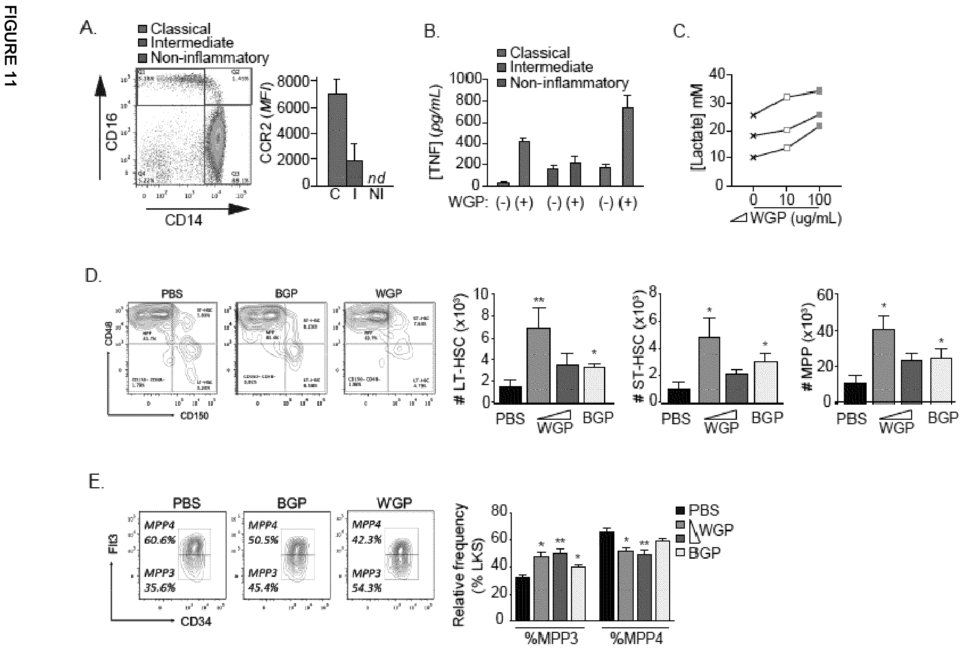

**Figure 11****.** Wellmune^{®} dispersible (dWGP) responses in primary human monocytes, mouse BMDM and mouse bone marrow. **A)** Monocytes were sorted based on CD14/CD16 expression as indicated (left panel) and subjected to further analysis based on CCR2 expression by flow cytometry (right panel). **B)** Monocytes isolated as in A were trained with dWGP for 24h and TNF production measured. **C)** 5-days post maturation BMDM were trained with dWGP (10-100 µg/mL) for 24h or left untrained. Lactate production was measured 5-days post-dWGP treatment. **D-E)** C57/BL6-JOIaHsd mice were injected intraperitoneally with dWGP (0.2 & 0.4 mg/mouse), BGP (1 mg/mouse) or vehicle PBS. After 7-days, bone-marrow (BM) was isolated, quantified, stained and analyzed by flow cytometry for the indicated populations; Lin-x, c-ki, Sca1 (LKS), Long-Term Hematopoietic Stem Cells (LT-HSC), Short-Term HSCs (ST-HSC), Multipotent Progenitors 1-3 (MPP) or CD150-/CD48- cells. Gating strategies for identification of LT-HSC, ST-HSC and MPP are shown in C) (left panel) alongside absolute numbers for each population. Gating strategies for identification of MPP subsets is shown in D) (left panel) alongside absolute numbers for each population. Data in A is mean MFI ± sem for n=3 donors. Data in B-C is mean value ± sem for n=3 independent experiments. Flow panels for representative samples are shown in C-D alongside mean total populations ± sem for n=4-5 mice per group.

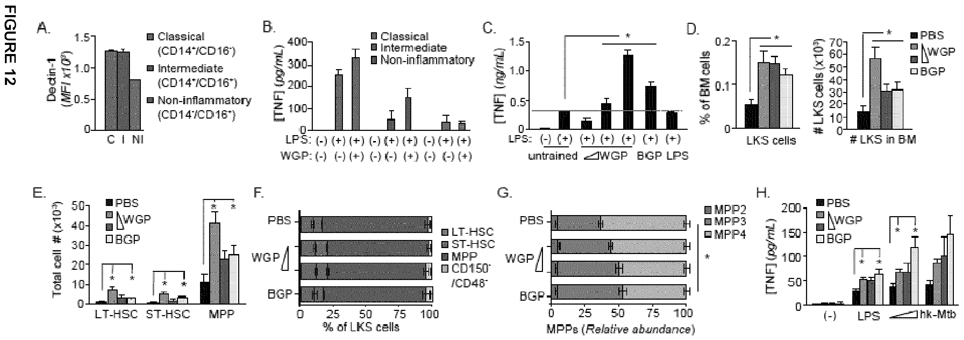

**Figure 12****.** Wellmune^{®} dispersible (dWGP) primes monocytes and drives myeloid bone marrow reprogramming. **A-B)** Monocytes were sorted based on CD14/CD16 expression as indicated and subjected to further analysis based on Dectin-1 expression by flow cytometry (A) or trained with dWGP, allowed to recover and restimulated with LPS (10 ng/mL, 6h, B). C) 5-days post maturation BMDM were trained with the dWGP (1,10-100 µg/mL), BGP (10 µg/mL) or LPS (5 ng/mL) for 24h. 11-days post-isolation mature macrophages were restimulated with LPS (10 ng/mL) and TNF production measured. **D-G)** C57/BL6-JOIaHsd mice were injected intraperitoneally with dWGP (0.2 & 0.4 mg/mouse), BGP (1 mg/mouse) or vehicle PBS. After 7-days, bone-marrow (BM) was isolated, quantified, stained and analyzed by flow cytometry for the indicated populations; Lin-x, c-kit, Sca1 (LKS), Long-Term Hematopoietic Stem Cells (LT-HSC), Short-Term HSCs (ST-HSC), Multipotent Progenitors 1-3 (MPP) or CD150-/CD48- cells. **H)** Splenic macrophages were isolated by plastic adherence and stimulated with LPS (10 ng/mL) or heat-killed *Mycobacterium tuberculosis* H37Ra (hk-Mtb, 500-1000 µg/mL) for 6h. Data in A is mean MFI ± sem for n=6 donors. Data in B-C is mean TNF value ± sem for n=3 independent experiments. Data in D is % LKS cells in total BM cells (left panel) or absolute LKS cell number (right panel). Data in E is absolute cell number for the indicated subset. Data in F is relative percentage for each subset in the total LKS population. Data in G is relative frequency for each population among total MPP pool, excluding MPP1 (gated separately, **see** **Figure 11**). Data in H is mean TNF value. All data in D-H is mean ± sem for n=4-5 mice per group.

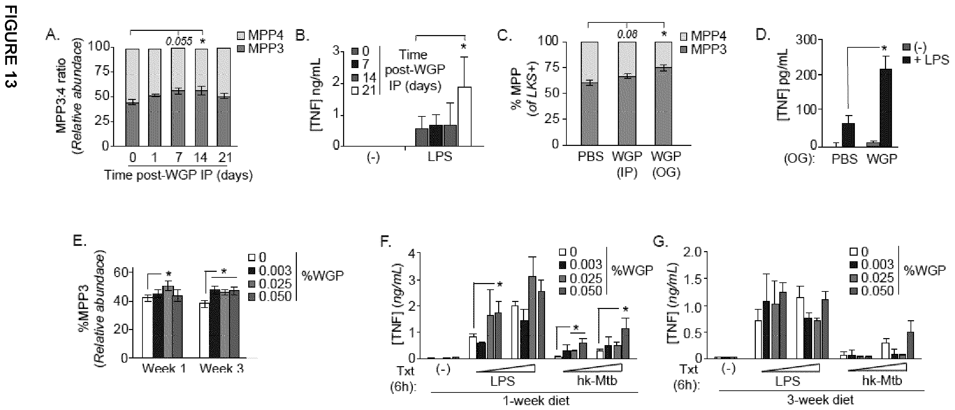

**Figure 13****.** Oral Wellmune^{®} dispersible (dWGP) reprograms bone-marrow macrophages responses. **A-G)** Mature C57/BL6-JOIaHsd mice were fed control (0 dWGP diet) for 2-weeks prior to initiation of feeding diets supplemented with increasing concentrations of dWGP (0, 0.003%, 0.025%, 0.050% per kg chow). Mice were sacrificed 1 or 3-weeks post-dWGP feeding and bone-marrow (BM) examined by flow cytometry (A, C and E) or matured to BMDM and stimulated with LPS (10 ng/mL, B and F-G) or heat-killed *Mycobacterium tuberculosis* H37Ra (hk-Mtb, 500-1000 µg/mL, F-G) for 6h and TNF measured by ELISA. Data in A) is absolute LKS cell number in total BM cells. Data in B) is relative percentage for each subset in the total LKS population. Data in C) is relative frequency of MPP3 population in the total MPP3/4 population. All data in A, C and E is mean ± sem for n=6 mice per group. Data in B, D and F-G is mean TNF value ± sem for n=4 mice per group.

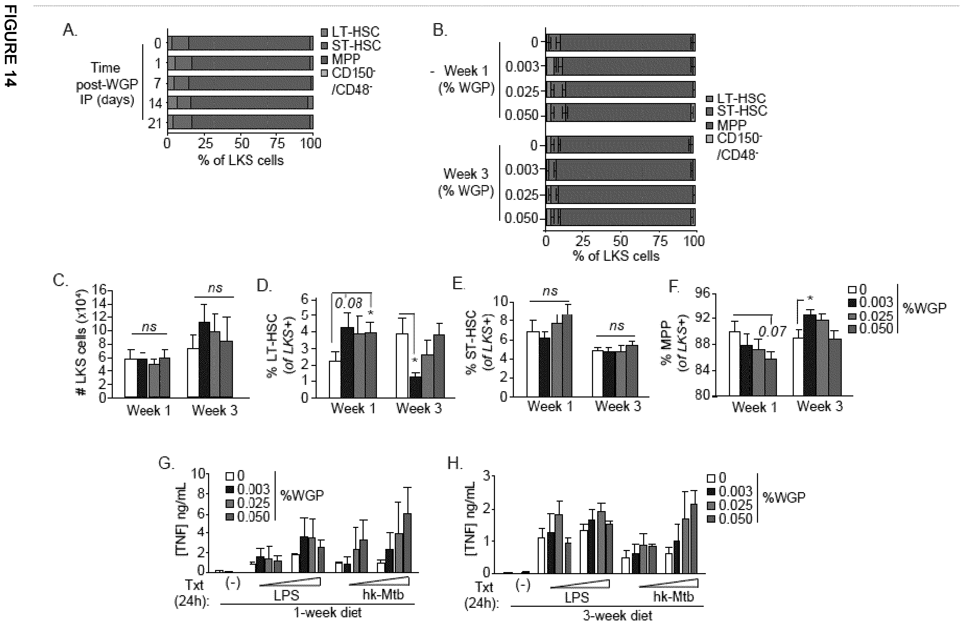

**Figure 14****.** Effect of oral Wellmune^{®} dispersible (dWGP) on bone-marrow HSPC profiles. **AH)** Mature C57/BL6-JOIaHsd mice were fed control (0 dWGP diet) for 2-weeks prior to initiation of feeding diets supplemented with increasing concentrations of dWGP (0, 0.003%, 0.025%, 0.050% per kg chow). Mice were sacrificed 1 or 3-weeks post-dWGP feeding and bone-marrow (BM) examined by flow cytometry (A-F) or matured to BMDM and stimulated with LPS (10 ng/mL) or heat-killed *Mycobacterium tuberculosis* H37Ra (hk-Mtb, 500-1000 µg/mL) for 6h and TNF measured by ELISA (G-H). Data in A) is relative percentage for the indicated subset in the total LKS population. Data in C) is relative frequency of MPP3 or MPP4 population in the total MPP3/4 population. Data in A-F) is mean ± sem for n=6 mice per group. Data in G-H) is mean TNF value ± sem for n=4 mice per group.

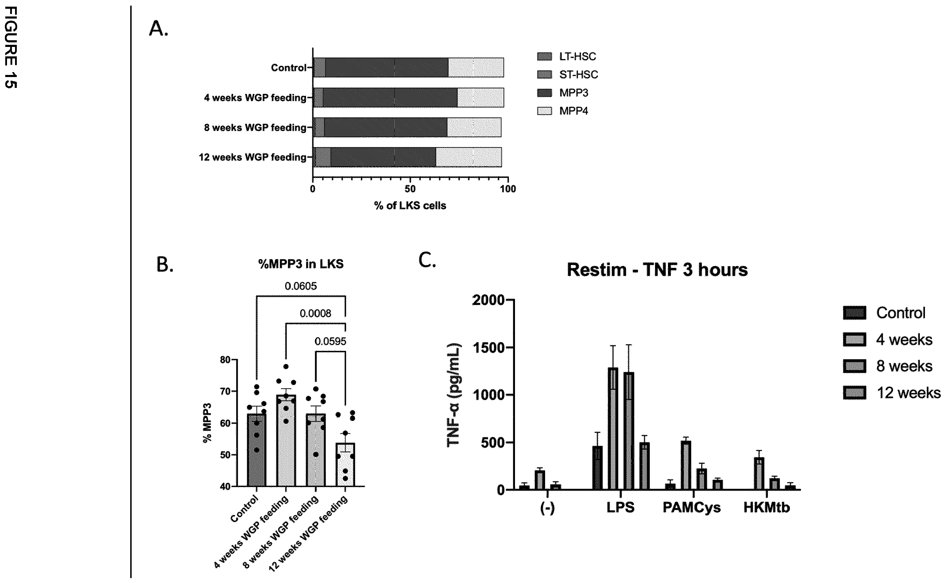

**Figure 15****.** Long term feeding of Wellmune reveals a transient increase in Trained Immunity. Mature C57/BL6-JOIaHsd mice were fed a diet supplemented with 0.05% dWGP per kg chow for 12, 8 or 4 weeks and compared to a 12-week no WGP control. Mice were sacrificed and % of total bone-marrow LKS+ cells examined by flow cytometry (A) with MPP3 subset shown in B. Separately, bone marrow was matured to BMDM and stimulated with LPS (10 ng/mL), PAM3CYS (10ug/mL) or heat-killed *Mycobacterium tuberculosis* H37Ra (hk-Mtb, 500µg/mL) for 3h and TNF production measured by ELISA (C). Data in A-B) is relative percentage for the indicated subset in the total LKS population. Data in C) is mean TNF value ± sem for n=5 mice per group

### Detailed Description of the Invention

The current invention provides an effective amount of a *Saccharomyces cerevisiae*-derived (1→3)-β-D-glucan for use in a method to programme bone marrow hematopoietic stem and progenitor cells (HSPC) in a subject. The HSPC in said subject provide multipotent progenitor (MPPs) expansion in favour of myelopoiesis., i.e., the production of innate immune cells, in particular monocytes, after exposure. The mature cells derived from this expanded population have an altered function.

Thus, the subject produces more MPP 3, i.e., myeloid precursors. Typically, the ratio of MPP4 to MPP3 in a subject is around 60:40 (Figure 12G). However, administration of the glucan of the invention causes the subjects HSPC to provide a greater MPP3 population and increase the ratio in favor of MPP3. The ratio may be 50 or 40:60 or 30: 70. It will be appreciated that this may depend on the condition, dose and/or subject.

Without being bound by theory, it is thought that the β-glucan elicits its effect via phagocytic C-type lectin receptor, Dectin-1 (encoded by *Clec7a*) (Brown, G. D. et al. Dectin-1 mediates the biological effects of beta-glucans. J Exp Med 197, (2003)) to drive metabolic and epigenetic changes in innate immune cells, in particular monocytes, which promotes enhanced macrophage response to restimulation, i.e., trained immunity.

Training of innate immune cells via epigenetic modification leads to enhanced innate immune gene induction causing a quantitively larger and earlier cytokine response, i.e., a generalized boost in immune signaling. It provides enhanced induction of chemokines by monocyte derived macrophages. It provides enhanced induction of pro-inflammatory cytokines (TNF). It provides enhanced anti-inflammatory immune regulatory (IL-10).

The inventors have shown that the whole fungal particle is recognized by Dectin-1 and internalized by the phagocytic synapse, thereby inducing an optimal anti-microbial response. Dectin-1 has signaling functions and has been shown to activate NFκB and pro-inflammatory gene expression through SYK/CARD10. Different β-glucans can engage Dectin-1 differentially depending on the mode of presentation. While soluble low molecular weight (MW) β-glucans can bind Dectin-1 and drive NFκB, recognition of larger β-glucan chains presented on intact fungal particles, such as the β-glucan of the invention, is required to drive surface Dectin-1 receptor localization and formation of the phagocytic synapse linked to anti-microbial activities like ROS.

β-glucan are commonly used and tolerated food ingredients and so are particularly suitable as a food supplement. In use, when a subject consumes the β-glucan of the invention, e.g., as a food supplement or food product, the subject's HSPCs favour MPP3 and innate immune cell production with immunological memory, thereby enhancing the subject's response against infection with pathogens.

Interestingly, a key feature of trained innate immune cells is that their recall ability is not linked to the nature of the training stimuli, i.e., they can respond efficiently to restimulation with diverse, non-specific ligands.

The β-glucan of the invention may be formulated as a composition comprising the β-glucan of the invention. In an embodiment, the β-glucan of the invention is dispersed.

The β-glucan of the invention may be a dietary or food supplement. The supplement may be selected from the group comprising a beverage, including a "shot" or small drink portion, a bakery product, a dairy product, a snack product, powder product, powdered milk, confectionary product, yoghurt, breakfast cereal, a bread product, nutritional supplement, a sports nutritional supplement. The supplement may be a powder supplement incorporated into a food or beverage product.

The β-glucan of the invention may be formulated in a capsule or tablet form.

Notably, the β-glucan of the invention is a yeast whole glucan particle (WGP). A whole glucan particle is the further purified β-glucan spheres from yeast (Saccharomyces cerevisiae) cells based on alkali solubility and substantially retains the *in vivo* glucan morphology/three-dimensional structure. WGP is not soluble. Methods of preparing a WGP are known in the art. Exemplary methods are disclosed in the publications disclosing Wellmune^{®} herein and in the following study, Li B, et al. Yeast glucan particles activate murine resident macrophages to secrete proinflammatory cytokines via MyD88- and Syk kinase-dependent pathways. Clin Immunol. 2007 Aug;124(2):170-81). WGP differ from other beta-glucan preparations in terms of beta-glucan purity but importantly that they maintain the intact yeast cell wall shape in a non-viable ghost-form. The WGP of the invention is commercially available for research purposes and known to activate traditional innate immune signalling pathways (https://www.invivogen.com/wgp-dispersible; Goodridge HS. et al., 2011. Activation of the innate immune receptor Dectin-1 upon formation of a 'phagocytic synapse'. Nature. 472(7344):471-5.), but has not been described to activate trained immunity, particularly when administered orally.

In an embodiment, the particle has a particle size, i.e., diameter, of from about 1 micron to about 5 microns (or µm), from about 2 microns to about 4.5 microns, from about 2.5 microns to about 4 microns, or from about 3 to about 3.5 microns. This may be the average diameter. At least 80% to 99%, or >85% or 90%, of the particles may have a diameter in this range. Particle size is measured using a laser differentiation particle size analyzer and such methods are known in the art.

The glucan of the invention may be a preparation comprising a plurality of beta-glucans. The WGP in the preparation have an average size, or size distribution in which the average value is from 1 to 6 microns, preferably 2 to 4 microns.

The glucan is from or isolated from the cell wall of baker's yeast (*Saccharomyces cerevisiae).*

Notably, the glucan of the invention is Wellmune^{®}.

Wellmune^{®} is a dietary food, beverage, and supplement ingredient. It is an insoluble, large whole glucan particle (WGP) derived from the cell wall of baker's yeast (*Saccharomyces cerevisiae*) wherein the β-1,3/1,6-glucan is composed of a backbone of glucose molecules linked via unique chemical links β1,3 chains to which are attached to glucose chains via β 1,6 links. The Wellmune^{®} glucan may be as disclosed in US4810646, US4992540, US5037972, US5082936, US5028703, US5250436, and US5506124, each of which is incorporated herein by reference.

It will be appreciated that a person skilled in the art would be capable of determining an appropriate dose of the glucan or supplement comprising the glucan of the invention to administer without undue experimentation. The amount and the frequency are as best suited to the purpose. The frequency of application or administration can vary greatly, depending on the needs of each subject, with a recommendation of an application or administration range from once a month to ten times a day, preferably from once a week to four times a day, more preferably from three times a week to three times a day, even more preferably once or twice a day.

In one aspect of the invention there is provided an effective amount of the *Saccharomyces cerevisiae*-derived (1→3)-β-D-glucan of the invention to prevent, delay the onset, or reduce the severity of a disease or infection in said subject. Notably, the subject may be one that is susceptible to infection after certain events or at certain times. For instance, the subject may be a high-performance athlete who has an increased susceptibility to upper respiratory tract (URT) infections. Using the glucan of the invention as a supplement may decrease incidence of URT in the window of time where they have increased vulnerability (e.g., after marathons). It is considered that their innate immune system has been trained to resist these infections more effectively.

In an embodiment, the glucan of the invention is co-administered or used with one or more antigens to drive antigen specific immune memory.

It will be understood that the features of the embodiments of the invention may be combined in any combination.

The invention will now be described with reference to specific examples. These are merely exemplary and for illustrative purposes only: they are not intended to be limiting in any way to the scope of the monopoly claimed or to the invention described. These examples constitute the best mode currently contemplated for practicing the invention.

### EXAMPLES

### METHODOLOGY OVERVIEW

The current inventors examined the ability of the food grade yeast WGP of the invention alongside a panel of diverse β-glucans to drive trained responses in human monocyte-derived macrophages, alongside HEK-based reporter cell assays to study Dectin-1 signaling. The inventors also examined the ability of this WGP to promote bone-marrow myelopoiesis which has been linked to the long-term memory effects of trained immunity.

The first part of the study, presented as Figures 1-5, describes the *in vitro* innate immune Training of human monocytes. Leukocytes were isolated from healthy anonymous donors and monocytes isolated. These precursor cells were treated with training agents, e.g., S. *cerevisiae* Wellmune^{®} dispersible (dWGP) or controls for 24h, rested and allowed to mature to macrophages. After this rest period, mature macrophages were re-stimulated with common innate immune activators, mainly the gram-negative component LPS, and cytokine production measured using a TNF ELISA of supernatants from these cultures. Training is indicated by an enhanced response to LPS restimulation, measured by increased TNF production in dWGP-trained cells over control untrained.

In the second part of the study, bone-marrow was taken from all mice and grown to mature macrophages (BMDM) in the lab using L929-conditioned media. Upon maturity (6 days post differentiation), BMDM were stimulated with microbial agonists at various doses eg - lipopolysaccharide (LPS) of *E. coli* cells or heat-killed *Mycobacterium tuberculosis* (Mtb) which activates the BMDM to produce proinflammatory cytokines e.g., TNF. This acts as a readout of innate immune function enhanced by trained immunity.

### MATERIALS AND METHODS

### Detailed Methods:

### Cell isolation and culture reagents

hDectin1b-HEK293 NFκB-SEAP reporter cells were obtained from Invivogen and were cultured according to manufacturer's instructions. Puromycin and HEK-Blue^{™} CLR Selection (Invivogen) were used as selective antibiotics and cells were cultured using DMEM (4.5 g/L glucose), 10% (v/v) fetal bovine serum (FBS), 100 U/ml penicillin, 100 µg/ml streptomycin, 100 µg/ml Normocin and 2 mM L-glutamine. Human peripheral blood mononuclear cells (PBMCs) were isolated from buffy coats obtained from the Irish Blood Transfusion Services (Dublin, Ireland) using density gradient centrifugation with Lymphoprep (Stem Cell Technologies) followed by red blood cell lysis using Lysis Buffer Hybri-Max^{™} (Sigma-Aldrich). PBMCs were resuspended in differentiation media (cRMPI, 10% FBS, 10 ng/mL M-CSF) and monocytes enriched by adherence to plastic and used for subsequent training assays or stimulations. Supply of human blood products from IBTS was approved by clinical indemnity. Bone marrow derived macrophages (BMDM) were isolated by flushing the tibia and femur from both legs of C57BL/6J male mice generated and maintained at the Comparative Medicine Unit, Trinity College Dublin (Dublin, Ireland) with DMEM (Gibco). The suspension of bone marrow cells obtained was strained on a 40 µm nylon mesh cell retainer (Biolegend). After a wash, the pellet was resuspended and treated 2 minutes with Red Blood Cell (RBC) Lysis Buffer Hybri-Max^{™} (Sigma-Aldrich) to lyse erythrocytes. After washing and counting, cells were resuspended in DMEM, 10% FBS, 20% L929-conditioned media and seeded to be differentiated into BMDMs over 1 week. Mature BMDM were scraped and cells reseeded in the required density in DMEM, 10% FBS, 5% L929-conditioned media and allowed to rest overnight before stimulation.

### β-glucan preparations and training stimuli

*Saccharomyces cerevisiae*-derived whole glucan particles were provided by Dr Sonja Nodland, Kerry Health & Nutrition, Minnesota, USA. These include Wellmune^{®} whole-glucan particles (WGP) in which cell wall β-glucan was preserved - yielding non-aggregated ghost yeast cells - or a soluble preparation, derived from heat-treated WGP. For all experiments, WGP powder was resuspended in PBS and sonicated to de-clump and obtain dispersible "single-cell" ghost particles or dispersible WGP (dWGP). Although the soluble preparation consists of a heterogenous mixture of soluble β-glucans of varying molecular weights, we have referred to this formulation as soluble WGP (sWGP) to highlight that dWGP and sWGP are composed of the β-glucans from the same source. Dr Nodland also provided β-glucan fractions of differing molecular weights that were isolated from sWGP by size exclusion chromatography, followed by filtration (1.5-100 nm filtration steps). The fractions were as follows: F1: <100 kDa, F2: 100-400 kDa, F3: 400-800 kDa and F4: >800 kDa. The sonication procedure to generate dWGP from WGP powder was was as follows: WGP was weighed and dissolved in sterile endotoxin-free water (Invitrogen) to yield 10-15 mL of 25 mg/mL WGP. This solution was left at room temperature overnight (8-16 h) before sonication with a 150VT ultra sonic homogenizer with a 5/32" microtip. The solution was sonicated for 5 min, at 50% power and 50% time pulse rate, while the tip was immersed roughly 5 mm below the surface of the liquid. Due to the heat caused by the sonication, the tube containing the solution was kept in ice. Following this sonication step, the dWGP was pelleted via centrifugation (1,000 G, 10 mins, at room temperature) and the water was removed by careful decanting and replaced with 0.2 M NaOH in water at a volume to reach 25 mg/mL WGP. After 20 minutes, the dWGP was washed three times with sterile water, using the same pelleting, decanting and replacement of solvent conditions as described. Finally, two last washes were carried out to replace the sterile water with sterile PBS and stored at 4 °C for up to 6 months. As dWGP settles out of solution, it also required vortexing prior to each use. Unsonicated whole-glucan particles (uWGP) was used in some experiments and was resuspended directly in PBS without sonicating.

For some experiments, 3 µm aminated polystyrene particles (AM-PS; Magsphere) were conjugated with sWGP, based on a previously published method (Tam J. M. et al. Use of fungal derived polysaccharide-conjugated particles to probe Dectin-1 responses in innate immunity. Integr Biol (Camb) 4, 220-227 (2011). 2 mg of AM-PS were washed with anhydrous DMSO (Sigma) three times, using centrifugal filters containing 0.65 µm PVDF membrane (Ultrafree, Millipore) before incubation with 250 µL of 2 M 1,1'-Carbonyldiimidazole (CDI; Sigma), freshly dissolved in anhydrous DMSO, for one hour at room temperature, whilst rocking. The particles were then washed twice with anhydrous DMSO to remove excess CDI (using again the centrifugal filters), prior to incubation with 250 µL of 0.1 mg/mL sWGP, dissolved in anhydrous DMSO, for one hour at room temperature, whilst rocking. Following this conjugation step, the sWGP was collected by centrifugation, using centrifugal filters to block the particles, and the efficiency of conjugation was assessed by measuring the loss of sWGP, using filtered sWGP solutions without AM-PS, or CDI, as controls. The sWGP was measured using a phenol sulphuric acid method, based on a reported protocol (Masuko, T. Carbohydrate analysis by a phenol-sulfuric acid method in microplate format. Anal Biochem. 339, 69-72 (2005). On a 96-well plate, 130 µL of concentrated sulfuric acid (Sigma) was added to 50 µL of sWGP sample, after which 30 µL of 5% w/v phenol (Sigma) in sterile water was added, followed by rapid pipetting up and down to mix the solution thoroughly. The plate was placed on a 90 °C hot plate for 5 min, then cooled to room temperature with a water bath. Absorbance of 492 nm was measured and a standard curve of known amount of sWGP was used to determine the concentration of sWGP in each sample, thereby quantifying how much sWGP had been conjugated onto the AM-PS.

Other β-glucans were obtained from Invivogen and include; Beta-glucan peptide (BGP) a high molecular weight polysaccharide extracted from the macrofungus *Trametes versicolor,* Zymosan, a *Saccharomyces cerevisiae*-derived cell wall preparation, Schizophyllan, a high molecular weight β-glucan derived from the fungus *Schizophyllum commune*, Curdlan, a β-1,3 linked glucan derived from the bacteria *Alcaligenes faecalis* and Pustulan, a median molecular weight linear β-1,6 linked glucan from the algal lichen *Lasallia pustulata.* All β-glucans were used at concentrations ranging 1, 10 or 100 µg/mL. Heat-killed Candida albicans (HKCA) was also obtained from Invivogen and used at concentrations between 1×10⁴⁻⁶ cells/mL.

### Macrophage activation/restimulations

Ultrapure lipopolysaccharide (LPS) from *E.coli* O111:B4 was obtained from Invivogen and used to induce tolerance in human monocytes at concentrations of 1, 10 and 100 ng/mL or used to restimulate trained macrophages at 10 ng/mL in most experiments or between 10 and 100 ng/mL. Pam3CSK4, a synthetic triacylated lipopeptide TLR2/TLR1 agonist was obtained from Invivogen and used for restimulation at 100 µg/mL. HKCA was used to restimulate trained macrophages at 1×10⁶ cell/mL. Trained monocyte-derived macrophages were restimulated with non-viable irradiated *Mycobacterium tuberculosis* (iMtb) obtained from the American Type Culture Collection (ATCC) (Manassas, VA) and prepared according to manufacturer's instructions and used at 500 µg/mL. BMDM from *in vivo* trained mice were stimulated with heat-killed *Mycobacterium tuberculosis* (hk-Mtb) from Invivogen and used at concentrations between 500 and 1000 µg/mL. Trained monocyte-derived macrophages were also infected with viable *Mycobacterium tuberculosis* (Mtb) strain H37Ra also obtained from ATCC and propagated in Middlebrook 7H9 medium to log phase. On the day of infection, bacteria in log-phase were pelleted by centrifugation and resuspended in DMEM. Bacterial pellets were de-clumped by passing through a syringe with an 25G needle several times. A single cell suspension was isolated by centrifuging the bacterial suspension at 800 rpm for 3 min. The supernatant of this spin was quantified by spectrophotometry (OD₆₀₀ₙₘ) and used to infect macrophages. Macrophages were infected at an MOI of 5 bacilli per cell for 3h (as previously described by Hackett EE, et al., Mycobacterium tuberculosis Limits Host Glycolysis and IL-1β by Restriction of PFK-M via MicroRNA-21. Cell Rep. 2020 Jan 7;30(1):124-136.

purified further by centrifugation at 13,000 rpm for 10 min to pellet extracellular bacteria. Bacteria-free media was returned to macrophages after washing in DMEM to remove extracellular bacteria and cultures grown up to 72h post-infection.

### Training Assays & Readouts

For human monocyte training assays, PBMCs isolated from human blood were seeded in 96 well plates (100,000 cells per well in 180 µL RMPI, 10% FBS, 50 ng/mL M-CSF) and 20 µL of the training stimulus was added immediately. Cells were incubated overnight at 37°C. Media was removed and cells were very gently washed twice with 100 µL of warm PBS to remove training stimulus and 200 µL of fresh media was added. Cells were washed and given fresh media every 2-3 days and on day 5 cells were restimulated in fresh media for the indicated times. For training inhibition assays, PBMC cells were incubated with the desired inhibitor for the indicated time prior to the addition of the training stimulus. Inhibitor concentrations were as follows unless specifically indicated: 1 mM 5'methylthioadenosine, an excess of sWGP (10-100 µg/mL), 10-100 µg/mL laminarin (Invivogen), piceathanol between 4, 10 and 30 µM, 5 µM BAY-11087, between 1, 2.5, 5 and 10 mM 2-deoxyglucose, between 1, 10 and 100 nM Rapamycin, between 0.1, 1 and 10 uM wortmannin, between 2, 10 and 100 µM Cytochalasin D and between 1, 5 and 10 µM bafilomycin A1. All inhibitors were from Sigma-Aldrich unless otherwise indicated. As a readout of training, TNF secretion from trained cells was measured by ELISA of supernatants (human TNF ELISA kit, Invitrogen). Alternatively, CXCL8 or IL-10 production was measured by ELISA. For metabolic analysis of trained cells, lactate concentration was measured in supernatants using the colorimetric Lactate Assay Kit (MAK064, Sigma-Aldrich). Media removed from trained monocytes after 24h was also analyzed for TNF production using ELISA to assess the impact of training stimuli on monocyte activation or used to measure extracellular Lactate production. In some experiments, RNA was isolated from trained monocytes with the RNeasy Kit (Qiagen). For analysis of gene expression, cDNA was prepared with the High-Capacity cDNA Archive kit according to manufacturers' instructions (Applied Biosystems) and individual mRNAs were monitored with the following inventoried human TaqMan assays (Applied Biosystems): *18s* (Hs03003631_g1), hexokinase-2 (HK2, Hs00606086_m1), GLUT-1 transporter (*SLC2A1,* Hs00892681_m1), lactate dehydrogenase A (LdhA, Hs01378790_g1) and PKM-2 (Hs00987255_m1). The AB7900HT platform (Applied Biosystems) was used for all PCR, performed in triplicate in FAST mode. Changes in expression were calculated by the change in threshold (ΔΔCT) method with 18S as an endogenous control and were normalized to results obtained in untreated cells. For experiments where trained macrophages were infected with *Mycobacterium tuberculosis* (Mtb), baseline growth was assessed by lysing 3 h time-point in 0.1% Triton-X for 10 min. Serial dilutions were plated on 7H10 Middlebrook Agar in triplicate and colony-forming units enumerated after incubation at 37°C for 14-21 days after plating. For later growth measurements this lysate was combined with pelleted extracellular bacteria, obtained by centrifugation of supernatant and fold-change in bacterial colony forming units (CFUs) expressed relative to baseline time-point. For supernatant transfer experiments, supernatants from human monocytes 24h post-training were harvested and administered to naive, untrained monocytes alongside dWGP-trained monocytes, matured for 5-days prior to restimulation with LPS to assess if soluble factors induced by training stimuli conferred enhanced responsiveness to restimulation.

BMDM Training Assays were performed by stimulating BMDM with training stimuli 6-day post-isolation and allowing to recover and mature for a further 6-days in DMEM, 10% FBS, 5-7% L929-conditioned media, changing the media every 3 days prior to restimulation with LPS 12-days post-isolation. TNF production in supernatants was analysed using Invitrogen murine TNF ELISA kit as per manufacturer's instructions or Lactate production measured as above.

### QuantiBlue Assays

hDectin1b-HEK293 NFκB-SEAP reporter cells (Invivogen) were cultured according to manufacturer's instructions. Reporter cell assays were carried out by seeding at 50,000 cells per well in 180 µL in a 96 well flat-bottomed plate and incubating the cells with 20 µL of the indicated agonists overnight. SEAP activity was measured using QUANTI-Blue (Invivogen) according to the manufacturer's instructions. Briefly, 20 µL of supernatant was added to 180 µL of QUANTI-Blue solution and incubated at 37°C for 15 minutes. Optical density at 620 nm was then measured using a plate reader.

### Animal work

For *in vivo* induction of trained immunity, C57BL/6J-OlaHsd male mice were generated and maintained at the Comparative Medicine Unit, Trinity College Dublin (Dublin, Ireland). Mice were bred and maintained under specific pathogen-free conditions with *ad libitum* access to food and water. Mice were used at the age of 8-12 weeks. All experiments were carried out under the approval of the Health Products Regulatory Authority, Ireland and Trinity College Dublin Animal Research Ethics Committee. Trained immunity was induced in mice with a single intraperitoneal injection of either 200 µL of PBS as control or 200 µL of dWGP resuspended in PBS at 1 mg/ml or 2 mg/ml. For *in vivo* induction of trained immunity by oral supplementation, mice were split into four main experimental groups. The control group was fed a slightly modified standard diet containing 25 g/kg of inulin (2.5% inulin per kg chow) as a source of fibre (Ref. D11112201). The three other groups of mice were fed a diet supplemented with 0.003% (0.03 g per kg chow), 0.025% (0.25 g per kg chow) or 0.05% (0.5 g per kg chow) of the dietary fibre dWGP respectively, including a proportionally reduced amount of inulin to balance the amount of dWGP fibre incorporated in the supplemented diets. All groups of mice were allowed two weeks to habituate to the inulin-enriched standard diet before being switched to the dWGP-supplemented diets for 1 to 3 weeks. At the experiment's endpoint, animals were euthanised by CO₂ inhalation and tissues collected for analysis. Bone marrow cells were harvested by flushing the tibia and femur from both legs with DMEM (Gibco). Bone marrow progenitors were stained for flow cytometry analysis as described below or used to generate BMDM as previously described. After red blood cell lysis, washing and counting, 3 million cells were kept for flow cytometry analysis as described below, and the remaining cells were resuspended in DMEM, 10% FBS, 20% L929-conditioned media and seeded to be differentiated into BMDMs over 1 week. To isolate splenocytes, spleens were carefully dissected after euthanasia, collected and kept on ice in RPMI/FBS 0.1% medium. Spleens were then minced into small pieces, gently crushed through a 70 µm nylon mesh cell retainer and diluted with PBS. After a wash, the pellet was resuspended and treated 2 minutes with Red Blood Cell (RBC) Lysis Buffer Hybri-Max^{™} (Sigma-Aldrich). Splenocytes were then washed again, resuspended in DMEM, 10% FBS, 5% L929-conditioned media and seeded. Cells were stimulated with LPS (10 ng/mL) or heat-killed *Mycobacterium tuberculosis* H37Ra (hk-Mtb, 500-1000 µg/mL).

### Multiparameter flow cytometry analysis of human monocytes

To analyse phospho-S6 ribosomal protein activity after β-glucan treatment, PBMCs were isolated as above and resuspended in RPMI-1640 media supplemented with 10% human AB serum (Sigma-Aldrich) at 2 × 10⁶/mL. Cells were first incubated with metabolic inhibitors for 15 min as indicated above. Cells were then stimulated for 30 min, 1h, 2h, 6h or 24h with either LPS (10 ng/mL, Invivogen), sWGP (10 µg/mL), uWGP (10 µg/mL) or dWGP (10 µg/mL). After the incubation time, cells were resuspended by vortexing quickly, washed in flow buffer; [PBS-1X (Gibco) supplemented with 5% heat inactivated FBS (Gibco) and 0.1% Sodium azide (Sigma)] and the following flow cytometry staining protocol was applied to all samples with up to 100,000 cells per sample. Cells were stained with fixable viability stain ZombieAquaTM (Biolegend) at the concentration of 1:500 for 15 minutes. Subsequently, samples were washed with flow buffer and incubated with anti-Dectin-1-PE (clone 15E2, Biolegend) antibody for Dectin-1 surface staining, then washed and fixed 20 minutes with IC Fixation Buffer (Invitrogen). Alternatively, cells were incubated with anti-CD14-APC (clone M5E2, Biolegend), anti-CD16-PE-Cy7 (clone 3G8, Biolegend), anti-HLA-DR-BB515 (clone G46-6, BD Bioscience) at a concentration of 1:100 in flow buffer for 30 minutes at 4°C. All cells were subsequently washed with flow buffer and resuspended 20 minutes with IC Fixation Buffer (Invitrogen) for fixation. Cells were then washed with flow buffer, incubated at 4°C for 30 minutes with Perm/Wash Buffer (BD Biosciences) containing anti-phospho-S6-PE (Ser235/236) (cloneD57.2.2E, Cell Signaling) at 1:200 for intracellular pS6 staining, and finally washed again with flow buffer. Compensation controls were obtained after staining UltraComp eBeads^{™} Compensation Beads (Invitrogen) with the appropriate antibodies. Cells were acquired on the BD flow cytometer Canto II with FACSDiva software. Data analysis and flow charts were performed using FlowJo software v.7.6 (TreeStar).

To sort human monocyte subsets, PBMCs were isolated from peripheral blood buffy coats as above. After isolation, PBMCs were resuspended in flow buffer and the following flow cytometry staining protocol was applied to all samples with up to 30,000,000 cells per donor. Cells were stained with viability stain Propidium Iodide (Biolegend) at the concentration of 1:500 for 15 minutes. Subsequently, samples were washed with flow buffer and incubated with anti-CD14-APC (clone M5E2, Biolegend), anti-CD16-PE-Cy7 (clone 3G8, Biolegend), anti-HLA-DR-BB515 (clone G46-6, BD Bioscience), anti-CCR2-APC-Cy7 (Biolegend) at a concentration of 1:100 in flow buffer for 30 minutes at 4°C. All cells were subsequently washed and resuspended in flow buffer. Compensation controls were obtained after staining UltraComp eBeads^{™} Compensation Beads (Invitrogen) with the appropriate antibodies. Cells were acquired and sorted on the BD FACSAria Fusion Cell Sorter with FACSDiva software. Data analysis and flow charts were performed using FlowJo software v.7.6 (TreeStar). After sorting, monocyte subsets were seeded separately in 96-well plates in RPMI-1640 media supplemented with 10% human AB serum (Sigma-Aldrich) at 1 × 10⁶/mL, left to rest for 24h, and subsequently stimulated and trained following the protocol described above.

### Multiparameter flow cytometry analysis of mouse bone marrow cells

To analyze HSPC populations in mouse bone marrow after *in vivo* induction of trained immunity, isolated bone marrow cells were resuspended in flow buffer [PBS-1X (Gibco) supplemented with 5% heat inactivated FBS (Gibco) and 0.1% Sodium azide (Sigma)] and the following flow cytometry staining protocol was applied to all bone marrow samples with up to 3,000,000 cells per sample. Cells were stained with fixable viability stain ZombieAqua^{™} (Biolegend) at the concentration of 1:500 for 15 minutes. Subsequently, samples were washed with flow buffer and incubated with anti-CD16/32 (Biolegend) at a concentration of 1:100 in flow buffer for 20 minutes at 4°C. The following antibodies were then used for staining Lin- c-Kit+ Sca-1+ cells (LKS), hematopoietic stem cells (HSCs) and multipotent progenitors (MPPs): anti-Ter-119, anti-CD11b (clone M1/70), anti-CD5 (clone 53-7.3), anti-CD4 (clone RM4-5), anti-CD8a (clone 53-6.7), anti-CD45R+ (clone RA3-6B3), anti-Ly6G/C+ (clone RB6-8C5), all biotin-conjugate (all Biolegend) were added at a concentration of 1:50 for 30 minutes at 4°C. Cells were then washed with flow buffer. Streptavidin - APC-Cy7 (Biolegend), anti-c-Kit-APC (clone 2B8, Biolegend), anti-Sca-1-PE-Cy7 (clone D7, eBioscience), anti-CD150 - eFluor450 (clone mShad150, eBioscience), anti-CD48-PerCP-eFluor710 (clone HM48-1, BD Bioscience), anti-CD34-FITC (clone RAM34, eBioscience), anti-Flt3-PE (cloneA2F10.1, Biolegend) were added and incubated at 4°C for 30 minutes. Fluorescence Minus One (FMO) controls were performed using 1,000,000 cells obtained by mixing equivalent volumes of samples coming from the different experimental conditions and stained with the proper antibodies. All cells were subsequently washed with flow buffer and resuspended with IC Fixation Buffer (Invitrogen). Compensation controls were obtained after staining UltraComp eBeads^{™} Compensation Beads (Invitrogen) with the appropriate antibodies. Cells were acquired on the BD flow cytometer Canto II with FACSDiva software. Data analysis and flow charts were performed using FlowJo software v.7.6 (TreeStar).

### Experimental design, statistical analysis and presentation

Data shown represents the mean data for experiments carried out on human monocytes/macrophages derived from the number of indicated independent donors, the numbers of indicated animals per groups for *in vitro* studies, or independent BMDM preparations for mouse *in vitro* studies. Data was analyzed by Graph Pad Prism or Excel and graphs annotated in Adobe Illustrator to generate Figures. ANOVA was carried out on multiparameter experiments with post-hoc tests to indicate significant differences between treatment groups or conditions and these were indicated in Figures.

### RESULTS AND CONCLUSION

In a preliminary screen, the inventors first tested a variety of commonly available β-glucans for their ability to drive canonical Dectin-1/ NFκB signaling using HEK-Dectin-1b over-expressing reporter cells. Well described Dectin-1 activators including S. *cerevisiae* Zymosan (a cell wall β-glucan preparation) and *T. versicolor-derived* β-glucan peptide (BGP) drive NFκB signaling in a dose-dependent fashion, as did similar concentrations of β-glucans derived from fungal (Schizophylan), bacterial (Curdlan) and lichen (Pustulan) sources (Fig. 1A). Wellmune^{®} dispersible, a particulate form of *S*. *cerevisiae* β-glucan, where the β-glucan layer of the yeast cell wall is isolated and intact forming "ghost cells", referred to here as a dispersible whole-glucan particle (dWGP), also drove NFκB activation through Dectin-1b. However, a derived soluble form of the same *S*. *cerevisiae* β-glucan - referred to here as soluble WGP (sWGP), prepared by heat degradation of dWGP, resulting in a heterogeneous mixture of β-glucan strands of various molecular weights and which lacks the intact single cell structure of dWGP - did not drive NFκB activation through Dectin-1b.

The same β-glucan concentrations were then tested for their ability to induce monocyte training, by exposing freshly isolated human monocytes to these for 24h. After washing and maturing to human monocyte-derived macrophages (hMDM) for 5 days, cells were restimulated with the TLR4 ligand lipopolysaccharide (LPS) and extracellular TNF production measured as a readout of re-activation (Dominguez-Andres, J. et al. In vitro induction of trained immunity in adherent human monocytes. STAR Protoc 2, (2021)). Both BGP and LPS were used as respective positive and negative controls for macrophage restimulation (Fig. 1B, far left & far right) with dose-dependent exaggerated and impaired TNF responses observed indicative of both training and tolerance. A trend toward some training was observed with the other β-glucans including Curdlan at higher concentrations, while both zymosan and heat-killed C. *albicans* (HKCA) drove enhanced restimulation responses at lower concentrations tested. Significant training was observed in monocytes treated with higher concentrations of dWGP (10-100 µg/mL), while the soluble form of this β-glucan (sWGP) did not enhance response over control treated cells. Interestingly, the ability of various β-glucans tested to drive training, did not appear to be connected to their ability to drive canonical Dectin-1b NFκB signaling (Fig 1A and Fig. 2A). Therefore, the inventors decided to use the soluble synthetic peptide, with large molecular weight, BGP and ghost yeast cell particle dWGP and its soluble form sWGP in future studies to delineate the requirements for training.

As suggested by the Dectin-1b reporter studies, the ability of various β-glucan preparations to promote trained responses is not linked to their capacity to drive pro-inflammatory signaling at the training stage. BGP treatment at the monocyte phase (24h post-training) drove little TNF production, with more modest levels observed in dWGP-treated monocytes (Fig. 1C, right axis), despite the ability of both to promote similar macrophage training (Fig. 1C, left axis &. Fig. 2B). LPS, which drives a tolerance response in macrophages at the concentration(s) used, stimulates significant TNF production at the monocyte phase (Fig. 2C). On the other hand, BGP treatment at the monocyte stage increases viability of resulting macrophages (5 days post treatment,. Fig. 2D), while dWGP, sWGP or LPS treatment did not affect this.

The inventors therefore confirmed that dWGP β-glucan drove an inherent property within trained cells and found by transferring supernatants from monocytes trained with dWGP for 24h to untrained monocytes, that they were unable to drive enhanced restimulation responses to LPS upon maturation (Fig. 2E), supporting that training is not mediated by soluble factors. However, the inhibition of intracellular DNA methyltransferases by pre-treating monocytes with the 5'methylthioadenosine (MTA) prior to β-glucan training, was able to block the enhanced responsiveness of β-glucan-trained macrophages to both BGP and dWGP (Fig. 1D). This confirmed that these diverse β-glucans were training monocytes for enhanced responsiveness to restimulation as a result of epigenetic modification of cell fate.

Training of innate immune cells via epigenetic modification leads to enhanced innate immune gene induction, causing a quantitively larger and earlier cytokine response. dWGP-induced monocyte training led to increased TNF production after 3h and significantly at 6h post-restimulation relative to untrained macrophages, while LPS-tolerized macrophages display impaired TNF responses to restimulation (Fig. 3A). Similar results were seen with BGP-trained macrophages (Fig. 4A). While training has been shown to drive pro-inflammatory cytokine production, β-glucan-induced training led to a generalized boost in immune signaling, with both the induction of chemokines by macrophages (CXCL8), pro-inflammatory cytokines (TNF) and anti-inflammatory immune-regulatory cytokines (IL-10) enhanced by both dWGP and BGP training relative to control (Fig. 3B, Fig 4B). This indicates that trained cells respond faster and more efficiently yet still maintain regulation over their activation.

A key feature of trained innate immune cells is that their recall ability is not linked to the nature of the training stimuli, as they can respond efficiently to restimulation with diverse, non-specific ligands. The inventors confirmed this in dWGP-trained macrophages, with increased TNF production seen in macrophages restimulated with LPS as before, but also the TLR2 bacterial lipopeptide ligand PAM3CSK4 or when treated with heat-inactivated fungal *Candida albicans* (HKCA, Fig 3C). Similarly, challenge of trained macrophages with irradiated bacterial *Mycobacterium tuberculosis* H37Rv (iMtb) led to the increased production of a range of cytokines (TNF, IL-1β and IL-10) 24h post-treatment, while tolerizing with LPS dampened these responses (Fig 3D). The notion of enhancing early innate immune responses without triggering unregulated protracted inflammation is attractive to boost responses to infection, where impaired early containment can lead to exacerbated disease e.g. - COVID-19 and tuberculosis (TB). The inventors therefore tested the ability of dWGP-trained macrophages to contain intracellular infection. The inventors found superior containment in dWGP-trained macrophages after infection with Mtb H37Ra (72h) relative to untrained macrophages (Fig. 3E). Interestingly, although Mtb-infected dWGP-trained macrophages produce more TNF shortly after infection (Fig. 3F, 3h), levels normalize at later time-points (72h) when mycobacterial growth has been contained, supporting the notion that dWGP-trained macrophages possess a superior innate anti-microbial capacity.

After characterizing enhanced responses to restimulation in macrophages trained with dWGP, the current inventors sought to determine how such β-glucan particles like dWGP drive the training process in monocytes. Since both BGP and dWGP drove comparable levels of Dectin-1-mediated NFκB activation, the inventors examined if Dectin-1 is required for training downstream of these diverse β-glucans. It has previously been reported that soluble β-glucans can block activation of Dectin-1 signaling by larger β-glucan particles via occupying sites on the receptor and preventing binding and subsequent signaling. The inventors therefore employed Wellmune^{®} soluble (referred to here as sWGP) - which is a heterogenous mixture of high and low MW soluble β-glucans liberated from their particle structure - as well as laminarin, a soluble low MW β-glucan derived from macroalgae. Both compounds block Dectin-1b mediated NFκB activation in reporter cells driven by both BGP and dWGP, with sWGP slightly more effective in both cases (Fig. 5A). Pre-treatment of monocytes with sWGP prior to training was able to block the enhanced restimulation effect seen with multiple concentrations of dWGP (Fig. 6A) and similar results were obtained when laminarin and sWGP were compared side by side in both BGP and dWGP-trained cells (Fig. 5B), with laminarin more effective at all doses tested.

Thus, blocking Dectin-1 receptor occupancy can prevent monocyte training driven by larger MW β-glucans. Canonical Dectin-1 signaling uses the adapter protein SYK and although SYK-independent pathways exist, β-glucan-driven NFκB-activation is SYK-dependent. The inventors confirmed this in reporter cells by blocking NFκB activation driven by dWGP and BGP via pre-treatment with increasing concentrations of the SYK-kinase inhibitor picethanol (PIC, Fig. 5C). Similarly, PIC pre-treatment suppressed monocyte training driven by both BGP and dWGP as measured by LPS-restimulation responses (Fig. 6B), albeit it was more effective at higher concentrations than observed for NFκB activation. A similar result was observed when NFκB activation downstream of dWGP was blocked, using the IKKb inhibitor BAY 11-7082, with an abolishment of LPS-restimulation driven TNF production in trained macrophages (Fig. 6C). These data, taken alongside the earlier observation that while a range of β-glucans can activate NFκB, not all drive monocyte training, suggesting a model whereby early NFκB activation is required but not totally sufficient to reprogram myeloid responses to restimulation. Indeed, blocking early NFκB activation enhanced early TNF production, despite impairing training in dWGP-treated cells (Fig. 5D), suggesting a bifurcation in β-glucan/Dectin-1 responses linked to training. The inventors therefore sought to identify additional cellular pathways and processes impacted by β-glucan drivers of training.

*C*. *albica*ns derived β-glucan has been shown to drive significant metabolic reprogramming in trained monocytes which is linked to the epigenetic modifications required for enhanced macrophage responsiveness. In particular, up-regulation of cytosolic glycolysis driven by HIF1α emerged as a key signal activated through a Dectin-1/PI3K/mTOR pathway. The inventors measured the ability of a range of β-glucans to drive glycolysis in trained monocytes by measuring extracellular lactate production over time. The results show that dWGP is a strong inducer of this process that is maintained as monocytes differentiate over time (up to 6 days post-treatment), while similar soluble β-glucan preparations (BGP, sWGP) do not (Fig. 7A and Fig. 8A). Blocking the switch to glycolysis by targeting the gate-keeper enzyme hexokinase with 2DG abolished the ability of dWGP to increase LPS-restimulation responses (Fig. 7B). The inventors found that monocytes treated with dWGP drive phosphorylation of the mTOR substrate S6, in a rapamycin-dependent manner (Fig. 7C-E, gating strategy shown in Fig. 8B), indicative of mTOR activation. While LPS also drives this phenotype, treatment with the soluble sWGP does not. This up-regulated mTOR activity is particularly pronounced at early times post-treatment (2h, Fig. 8C), but is maintained up to 24h post-treatment when untreated monocytes upregulate mTOR as part of the normal differentiation process (Fig. 7F). This early mTOR activity promotes β-glucan training, since pre-treatment of dWGP-trained macrophages with rapamycin to block mTOR activity, limits trained responses to LPS-restimulation (Fig. 7G). mTOR activation downstream of Dectin-1 supports glycolytic reprogramming in trained monocytes by enhancing the expression of rate-limiting glycolytic genes like SLCA1, HK2, PKM2 and LDHA, which we observed in β-glucan trained cells 3-days after training (Fig. 7H). Previous data with C. *albicans* β-glucan has shown this glycolytic pathway is driven by PI3K signaling and indeed, addition of wortmannin at the training stage abolishes WGP-driven trained responses (Fig. 7I). Intriguingly, β-glucan driven Dectin-1 signaling leading to SYK activation was not required for mTOR activation, as pre-treatment of dWGP-trained monocytes with PIC did not affect mTOR up-regulation (Fig. 7J), despite its ability to block traditional pro-inflammatory signaling and monocyte TNF production (Fig. 8D). This data indicates a divergence of traditional Dectin-1 signaling and suggests an inherent property of particulate β-glucans may support the increased metabolic response required for training.

Previous comparisons of both soluble and particulate yeast β-glucans demonstrated that particulate structures drive anti-microbial responses, including phagocytosis through engagement of Dectin-1 clusters at the cell surface. Measuring cell surface Dectin-1 expression by flow cytometry, the inventors found that dWGP led to loss of Dectin-1 surface expression shortly after treatment (15 min), a trend not seen with the soluble BGP β-glucan (Fig. 9A, Fig. 10A). This loss of Dectin-1 surface expression by dWGP is dependent on phagocytosis since pre-treatment with the inhibitor of actin polymerization, cytochalasin-D (cytD) blunts this effect (Fig. 9B). Similarly, cytD pre-treatment prior to dWGP training, although affecting the ability of untrained cells to respond to LPS, preferentially blocked the training effect observed after LPS restimulation in dWGP-treated macrophages (Fig. 9C). CytD pre-treatment also blocked monocyte mTOR activation driven by dWGP (Fig. 9D). These data support a role for phagocytosis and internalization of dWGP-Dectin-1 receptor complexes to trigger mTOR and the subsequent metabolic response. β-glucan phagocytosis triggered by the internalization of bound-Dectin-1 clusters leads to phago-lysosomal fusion. The lysosome is also the reported site of mTOR activation in many other contexts, therefore the inventors hypothesized that β-glucan phagocytosis, preferentially driven by particles, delivers Dectin-1 complexes to the lysosome to engage the signaling machinery necessary to activate mTOR. Therefore, the inventors blocked lysosomal maturation using bafilomycin A1 (which blocks lysosomal membrane VATPase pumps required for acidification) and, similar to experiments with cytD pre-treatment, saw a profound decrease in monocyte mTOR activation by dWGP (Fig. 9E).

As soluble β-glucans do not trigger mTOR activation, lactate production or training in the same way, the inventors tested if other forms of yeast β-glucan particles could drive metabolic signaling and training. Preparation of Wellmune^{®} dispersible yeast ghost cells, consisting of intact β-glucan cell walls, can lead to crude collections of particles as clusters. These are disrupted by sonication to generate single dispersible particles, referred to as dWGP (illustrated in Fig. 10C). Unsonicated Wellmune^{®} dispersible preparations (uWGP) can drive NFκB activation in Dectin-1b reporter cells (Fig. 10D) and trigger significant early monocyte pro-inflammatory signaling as measured by TNF production 24h post-treatment, to a similar extent as observed with dWGP (Fig. 9F). However, uWGP treatment was not able to drive monocyte training as seen with dWGP and in fact, while dWGP training drove the characteristic enhanced TNF production to LPS-restimulation, uWGP-treated macrophages appeared impaired in terms of TNF responses to restimulation (Fig. 9G). Similarly, uWGP treatment of monocytes did not lead to a comparable loss of cell surface Dectin-1 expression as dWGP (Fig. 9H), suggesting these crude β-glucan clusters were too large to drive efficient phagocytosis. Consequently, uWGP-treated monocytes did not up-regulate extracellular lactate production (Fig. 9I), confirming the importance of Dectin-1/β-glucan complex internalization and phagocytosis to drive the metabolic reprogramming associated with training.

The insolubility of intact particulate β-glucans is determined by glucose chain lengths and linkages. Wellmune^{®} dispersible particles (dWGP) are a heterogenous complex of multiple cross-linked chain lengths of differing MW. The data suggests that size and preservation of β-glucan particles is a key property which controls recognition, uptake, internalization and engagement with the intracellular metabolic machinery. Thus, the inventor sought to determine the optimal chain length required for training. Although Wellmune^{®} soluble (sWGP), which cannot drive training, it represents a chemically identical form of the dWGP β-glucan where the β-glucan chains have been liberated from the yeast ghost cell to allow solubility. It represents a heterogenous mixture of glucan chains and MWs in a soluble, non-particulate form. The inventors thus isolated specific MW fractions of the sWGP preparation, such that each fraction represented pure β-glucan of varying chain lengths defined by their MWs and compared these to the dWGP and sWGP formulations in signaling studies. Although some of these fractions could drive NFκB activation in Dectin-1 reporter cells, notably the lower MW fraction (F1, >100 kDa), none of these drove training to the same extent as dWGP (Fig. 9J). Notably, these soluble yeast-derived β-glucans of defined MW enhanced LPS-restimulation responses when compared to the sWGP preparation (Fig. 9K) and taken with the finding of increased signaling capacity in Dectin-1b reporter cells, indicates that it is overall solubility determined by the extraction method rather than chain length and β-glucan MW which determines optimal training through Dectin-1.

Since soluble forms of yeast-derived β-glucan can drive NFκB activation but do not drive training, unlike the dWGP particle, the inventors hypothesized that particle recognition was a key step in training monocytes, ensuring appropriate activation of metabolic reprogramming. To test this, the inventors conjugated Wellmune^{®} soluble yeast β-glucan (sWGP) to 3 µm aminated polystyrene particles (AM-PS), shown in Supp. Fig. 10E by the loss of sWGP in the conjugated preparation. Human monocytes were then stimulated with the resulting particles or with controls: the equivalent amount of sWGP, AM-PS alone or dWGP. Comparing the increase in extracellular lactate production after 3 days, indicative of upregulated glycolysis, it was found that both sWGP alone and AM-PS particles alone did not alter cellular metabolism (Fig. 10L). However, sWGP conjugated to AM-PS gained the capacity to drive lactate production, albeit to a lower extent than pure dWGP particles (Fig. 9L). These data demonstrate that while β-glucan is required to activate traditional Dectin-1 signaling, the recognition of β-glucan presented on microbial-sized particles is required to trigger metabolic reprogramming and investment of the cell in a trained phenotype.

Much of the training literature has used *in vitro* stimulation of monocytes and therefore, to establish the relevance of the β-glucan/Dectin-1 pathway in humans in which various monocyte subsets exist, the inventors examined the expression of Dectin-1 across these cell populations. Compared to CCR2, a marker of inflammatory, migrating monocytes (Fig. 11A), Dectin-1 had lower overall expression but was present on classical inflammatory and intermediate macrophages, gated on CD14/CD16 expression. It is also present, albeit lower, on non-inflammatory CD14-/CD16+ monocytes (Fig. 12A). Despite all 3 subsets responding to initial treatment with WGP as measured by TNF production 24h post-stimulation (Fig. 11B), only Classical and Intermediate monocytes displayed enhanced LPS responses upon restimulation, indicative of training (Fig. 12B). Since the presence or absence of Dectin-1 on monocytes did not predict training, the inventors considered other cellular substrates for training *in vivo.*

HSPCs have emerged as sensitive to systemic delivery of training stimuli through various methods including intravenous delivery of BCG, IP injection of C. *albicans* β-glucan or hypercholesterolemia-induced NLRP3-inflammation driven by western diet feeding. In particular, the resulting inflammation leads to an increase in total bone marrow HSPC numbers with an increasing skewing of the ratio of multi-potent progenitors (MPP) toward myeloid-committed MPP3 and away from the more dominant lymphoid MPP4 cells.

The inventors determined if mouse bone-marrow derived macrophages were amenable to training: training with dWGP and similarly to BGP resulted in enhanced LPS restimulation responses in BMDM that had been trained 5-day prior to restimulation (Fig. 12C). This correlated with enhanced production of extracellular lactate by these cells (Fig. 11C), supporting a model whereby WGP reprograms mature mouse macrophages by metabolic reprogramming *in vitro.* The inventors determined if similar long-term immune training can be observed *in vivo* by examining HSPC progenitor cells in mouse bone-marrow. Importantly, mouse HSPCs express low levels of *Clec7a* mRNA, therefore changes in the bone-marrow compartment after systemic delivery of training agents by IP delivery were examined.

The inventors found that IP injection of WGP led to an increase in total bone-marrow c-Lin⁻, ckit⁺, Sca-1⁺ cells (LKS+) HSPC cells 1 week after administration (Fig. 12D), in a similar fashion to that previously observed with BGP injection, and this expansion was notably stronger with lower amounts of dWGP β-glucan. There was significant expansion in the absolute numbers of LT-HSPC and ST-HSPC (representative flow-charts shown alongside quantifications in Fig 11D), indicative of increased metabolic and proliferative activity required to supply HSPC turnover, as well as significant expansion in the more committed lineage MPPs (Fig. 12E-F) although the overall frequency of each subset was not significantly altered. However, looking within the MPP cells (gating shown alongside quantifications in Fig. 11E), which are the more abundant subset, the inventors observed both a marked expansion of myeloid-committed MPP3 cells at the expense of lymphoid-progenitor MPP4 in mice injected with both concentrations of dWGP employed, as observed with an equivalent high concentration of BGP (Fig. 12G). The abundance of megakaryocyte-linked progenitors MPP2 were not significantly altered across treatments. As well as increased myelopoiesis, training stimuli have been shown to reprogram HSPCs for enhanced activity upon maturation, consistent with what has been observed for peripheral monocyte training. Spleens were isolated to generate splenic macrophages shortly after sacrifice and these were stimulated with the TLR4 ligand LPS or various concentrations of heat-killed Mtb (hk-Mtb). Splenic macrophages from animals trained *in vivo* with BGP or the lower concentration of dWGP displayed significantly enhanced TNF production relative to splenocytes from control injected mice (Fig. 12H). These data indicate that IP delivery of particulate β-glucans can lead to mouse bone marrow reprogramming, quantitatively increased myeloid cell turnover and qualitatively alter their phenotype such that resulting mature cells have a heightened response to activation.

A major issue in the trained immunity field remains the longevity of trained effects. Therefore, the inventors conducted a time-course analysis of the effects on bone-marrow myelopoiesis after IP injection of dWGP. Consistent with earlier results, when C57/BL6-JOIaHsd mice were injected with 0.2 mg dWGP, a significant increase in the percentage of MPP3 cells, at the expense of MPP4 cells, 1-week post-injection was found (Fig. 13A). This was not seen 1-day after dWGP injection and was maintained up to 2-weeks post-injection, but resolved by 3-weeks. Importantly, splenocytes extracted from these mice displayed enhanced responses to LPS stimulation, particularly significant and maintained at 3 weeks post-injection (Fig. 13B). These results are consistent with earlier studies of β-glucan injection which demonstrated a dynamic remodeling of bone-marrow progenitors over time.

β-glucans represent a key class of non-digestible dietary fiber present in many foods and supplements. Yeast β-glucan's in particular are well tolerated and safe. The inventors analyzed if dietary consumption of Wellmune^{®} dispersible (dWGP) could drive training effects *in vivo.* A dose equivalent to the amount injected IP (0.2 mg dWGP) was delivered but through oral gavage (OG). Bone marrow was taken 1-week post OG and examined for HSPC subsets. Although an expansion of total HSPC numbers similar to that observed with previous IP injections was not observed by OG administration (data not shown), the ratio of myeloid-committed MPP3 cells did increase in mice given dWGP by both IP and OG routes (Fig. 13C). Splenocytes from these mice were stimulated with LPS and increased TNF production was observed from mice given dWGP via oral gavage (Fig. 13D). These qualitative and quantitative differences in bone marrow effects are likely as a result of different routes of administration of training agents, however this data supports the notion that consumption of β-glucans could enhance innate immune functions.

To examine this in a more relevant setting, the inventors designed a feeding study whereby increasing doses of dWGP, incorporated as the commercially available ingredient Wellmune, were fed to groups of mice alongside control diets enriched equivalently with an inert non-digestible dietary fiber (inulin) to match for dietary energy and fiber intake for up to 3 weeks. Again, an expansion in total bone-marrow LKS+ cell numbers in dWGP-fed mice did not occur (Fig. 14C), as observed in the prior OG study. Despite this, dynamic changes were observed in LKS populations overtime, with an increase in LT-HSC frequency in mice after 1-week of feeding which was followed by a consequent decrease in this population relative to control 3-weeks post-feeding, alongside increased percentages of more committed MPP cells at this same time-point (Fig. 14D-E). Crucially, the ratio of myeloid-committed MPP3 to lymphoid-committed MPP4 cells was significantly increased in mice fed dWGP-containing diets relative to control chow, particularly at 3-weeks after feeding began (Fig. 13E). Importantly, mature BMDM derived from the bone marrow of these mice displayed differences in functional responses to stimulation. BMDM generated from mice after 1-week of feeding displayed enhanced TNF production in response to stimulation with LPS or hk-Mtb, which was not apparent in BMDM taken from mice with 3-week feeding (Fig. 13F-G). In particular, cytokine production was enhanced in Wellmune^{®} dispersible-fed mice at 6h post-stimulation and were less apparent 24h-post stimulation (Figure 14G-H), consistent with the reprogramming effect of training enhancing kinetics of innate immunity. Notably, differences in cytokine production were more apparent in mice fed the highest amount of dWGP, whereas the effect on HSPC expansion was more apparent in mice fed lower amounts of dWGP, suggesting that the qualitative and quantitative effects on bone marrow reprogramming by training can be uncoupled and oral consumption of training reagents can differentially engage these. In conclusion, Wellmune^{®} dispersible is a yeast-derived β-glucan particle which can drive trained responses in innate immune cells - both monocytes and bone-marrow progenitors.

To do this, the inventors designed various mouse chow diets with increasing doses of dWGP, present as the trade-name "Wellmune" composition (Carpenter, K. C., et al., Baker's yeast beta-glucan supplementation increases monocytes and cytokines post-exercise: implications for infection risk? Br J Nutr 109, 478-486, (2013)), in increasing amounts and matched control diets with an inert non-digestible dietary fiber (inulin) to match for calorific consumption and roughage effects. C57/BL6OwaJ mice were fed these Wellmune-containing diets for up to 4 weeks and bone-marrow populations examined. Unlike BGP or dWGP-injected mice, the inventors did not observe an expansion in total bone-marrow LKS+ cell numbers in WGP-fed mice (Fig. 12A).

However, when the inventors examined the relative frequency of specific HSPC populations, the inventors did observe an increase in LT-HSC frequency in mice after 1-week of feeding which was followed by a consequent decrease in this population relative to control 3-weeks post-feeding, with increased percentages of more committed MPP cells at this same time-point (Fig. 12B). This data indicates that although the dramatic expansion of HSPC populations which manifests after inflammatory challenge (IP injection) does not occur in mice fed training agents, there still exists dynamic remodeling of HSPC progenitors in these mice. Although the ratio of myeloid-committed MPP3 to lymphoid-committed MPP4 cells did not significantly change (Fig. 12C, Fig. 13B), when the inventors generated mature BMDM from these bone-marrow we observed differences in functional responses to stimulation. BMDM generated from mice after 1-week of feeding displayed enhanced TNF production in response to stimulation with LPS or hk-Mtb, which was less obvious in BMDM taken from mice with 3-week feeding (Fig. 12D-E). In particular, differences in cytokine production were enhanced at 6h post-stimulation and were less apparent 24h-post stimulation (Figure 13C-D) consistent with the reprogramming effect of Training enhancing kinetics of innate immunity. Notably, differences in cytokine production were more apparent in mice fed the highest amount of WGP, whereas the subtle effect on HSPC expansion was more apparent in mice fed lower amounts of WGP, suggesting that the qualitative and quantitative effects on bone marrow reprogramming by training can be uncoupled and oral consumption of training reagents can differentially engage these. In conclusion, WGP is a yeast-derived β-glucan particle which can drive trained responses in innate immune cells - both monocytes and bone-marrow progenitors.

An additional feeding study was carried out in C57/BL6 mice fed a control diet (no Wellmune^{®} dispersible, dWGP) or the high Wellmune containing diet (0.05% dWGP) for between 0-12 weeks. Mice were sacrificed and bone-marrow extracted. The HSPC cell populations were measured by flow cytometry (Fig 15A-B) and displayed a marked expansion in the percentage of the myeloid-commited MPP3 population after 4-weeks of WGP feeding. This however was reduced to control levels after 12 weeks of feeding... Moreover, these bone-marrow cells were matured to BMDM and restimulated with TLR ligands. BMDM from mice-fed Wellmune displayed enhanced TNF production after restimulation in mice fed Wellmune for 4 & 8 weeks, but not in mice fed Wellmune for 12 weeks. This data is consistent with earlier data showing that Wellmune feeding although it does not drive expansion in HSPC numbers, does affect the frequency and turnover of HSPC cells in the bone-marrow and optimally at periods of 3-4 weeks, drives myeloid committed MPP3 skewing, indicative of central training in-vivo. This effect is not maintained after 8 -weeks of feeding.

Thus, mice consuming Wellmune via dietary supplementation display features of Trained Immunity with reprogramming of bone marrow HSPC populations over time and enhanced innate immune function in mature macrophages derived from these cells.

### DISCUSSION

The inventors uncovered a pathway whereby recognition of intact fungal particles and subsequent internalization via the phagocytic synapse is intimately linked to metabolic investment by the target cell into a trained phenotype through engaging mTOR. Although the studies used artificial β-glucan-rich particles to engage Dectin-1 in this way, it is tempting to speculate that this represents a conserved pathway by the innate immune system to avoid inappropriate and wasteful resources in long-term memory responses to soluble ligands or non-viable pathogens and rather to promote a trained phenotype only when necessary, in response to intact fungal particles. This involves reprogramming cellular metabolism to adopt a glycolytic profile only when lysosomal mTOR has been successfully engaged following phagocytosis of intact particles. This commitment to glycolysis is linked to the epigenetic changes which underline the trained phenotype and accelerated response to non-specific restimulation. Having observed this pathway in monocytes which are short-lived unless they migrate to tissues as macrophages, the inventors also observed the ability of artificial β-glucan-rich particles (dWGP) to affect myeloid progenitor cells in the bone-marrow as well as enhance functional responses in mature macrophages. These functional responses include cytokine responses. Thus, exploiting the physical and chemical requirements for optimal trained responses could be used to improve innate immune function for therapeutic approaches like vaccination or immunotherapies or to promote increased resistance against novel pathogens to which we have no pre-existing acquired memory, like the current scourge of COVID19.

Wellmune^{®} dispersible (dWGP) itself represents an artificial β-glucan preparation, rich in β-glucan which has had contaminating TLR2 ligands and other mannans removed. It has been observed in C. *albicans* that outer cell wall mannans block β-glucan interacting with the innate immune system and driving phagocytosis and thus mannans may have emerged as an immune evasion strategy to block education of the host by β-glucan exposure.

This work reveals that it is not the MW in β-glucan-chains in a mixture which affects their interaction with the innate immune system but ultimately the physical nature and way in which these ligands are presented to innate immune cells i.e., their insoluble nature, determined by the extraction process that preserves highly pure and intact ghost yeast cell walls. Soluble yeast β-glucans have been employed in both immunotherapeutic approaches and nutritional supplementation strategies. Although these data suggest Wellmune^{®} soluble cannot train and instead blocks receptor occupancy by higher MW particulate β-glucans, other soluble β-glucans can drive trained responses both *in vitro* and *in vivo,* including *Trametes versicolor* BGP. The mechanisms underlying this remain undefined although they may involve enhanced proliferation without significant glycolytic activity to epigenetically alter cell fate (Figs 12A), as has been shown in training models with C. *albicans* (β-1,3)-glucans or enhanced growth factor signaling linked to differentiation shown recently for other yeast β-glucan preparations.

The WGP employed here, sold as Wellmune^{®} dispersible, has been studied for its effect on upper respiratory tract infections in elite marathon runners as well as circulating cytokine levels and LPS-induced monocyte production in immunocompromised populations after consumption through oral route. The inventors' finding that oral consumption of WGP has biological and training-like affects in mice, consistent with *in vitro* training studies in human monocytes, opens up new avenues for exploitation of this class of molecule commercially and therapeutically. Notably, the effects on mouse bone-marrow myelopoiesis through the two distinct routes examined were discreet. Here the inventors show that WGP impacts bone marrow hematopoiesis by both quantitively increasing HSPCs and qualitatively skewing towards myelopoiesis.

Since WGP-trained macrophages display both increased clearance of Mtb and upregulated glycolytic metabolism it may be that metabolically reprogrammed trained macrophages are metabolically primed to be more hostile toward intracellular pathogen replication. Indeed, although WGP-trained macrophages produce more pro-inflammatory cytokines at early times post-infection with Mtb, by later times when the macrophages clear more Mtb, cytokine levels have normalized, supporting a notion that training does more than epigenetically prime and alter cytokine dynamics. The inventors' work places mTOR activation as central to training induced by β-glucan particulates. They demonstrate it is up-regulated by dWGP in a rapamycin-sensitive manner. S6K itself is an important ribosomal protein to drive protein synthesis activated by mTOR in response to changes in lysosomal activity and its activation during the training phase driven by dWGP, suggests that large scale proteomic and metabolic changes occur in trained macrophages which may persist throughout maturation.

In conclusion, the inventors identified a new immunomodulatory role for a yeast-derived particulate β-glucan through driving metabolic reprogramming in target cells required for trained immunity. This may affect strategies using these and similar β-glucans to promote innate immune function and may be useful in promoting innate immune resistance to infection.

## Claims

1. An effective amount of a *Saccharomyces cerevisiae*-derived (1→3)-β-D-glucan for use in a method to program bone marrow hematopoietic stem and progenitor cells (HSPC) to promote multipotent progenitor (MPPs) expansion in favour of myelopoiesis in a subject, wherein the (1→3)-β-D-glucan is a whole glucan particle, which is substantially spherical.

2. The effective amount of a *Saccharomyces cerevisiae*-derived (1→3)-β-D-glucan for use of Claim 1, wherein myelopoiesis produces trained innate immune cells.

3. The effective amount of a *Saccharomyces cerevisiae*-derived (1→3)-β-D-glucan for use of Claim 2, wherein the immune cell is a monocyte and/or a monocyte derived macrophage.

4. The effective amount of a *Saccharomyces cerevisiae*-derived (1→3)-β-D-glucan for use of any one of the preceding claims, formulated for oral administration.

5. The effective amount of a *Saccharomyces cerevisiae*-derived (1→3)-β-D-glucan for use of any one of the preceding claims, to prevent, delay the onset, or reduce the severity of a disease or infection in said subject.

6. The effective amount of a *Saccharomyces cerevisiae*-derived (1→3)-β-D-glucan for use of any one of the preceding claims, wherein the subject is in the early stages of a disease or infection, at risk of a disease or infection, or when the subject is suspected of having a disease or infection.

7. The effective amount of a *Saccharomyces cerevisiae*-derived (1→3)-β-D-glucan for use of any one of the preceding claims, wherein the subject is one or more selected from the group comprising, a healthy subject, an athlete, a subject experiencing or suffering from stress, an immunocompromised subject, a subject over 65 years of age, preferably over 75 years of age, a subject less than 16 years of age, a subject that has cancer or has recovered from cancer, and a subject with defective innate immunity.

8. The effective amount of a *Saccharomyces cerevisiae*-derived (1→3)-β-D-glucan for use of Claim 7, wherein the subject is a healthy subject.

9. The effective amount of a *Saccharomyces cerevisiae*-derived (1→3)-β-D-glucan for use of any one of the preceding claims, wherein the (1→3)-β-D-glucan is a β-1,6 branched β-1,3 glucan (or "β-(1,3/1,6)) or a β-1,4 branched β-1,3 glucan.

10. The effective amount of a *Saccharomyces cerevisiae*-derived (1→3)-β-D-glucan for use of Claim 9, wherein the (1→3)-β-D-glucan glucan is a β-1,6 branched β-1,3 glucan and has the following structure:

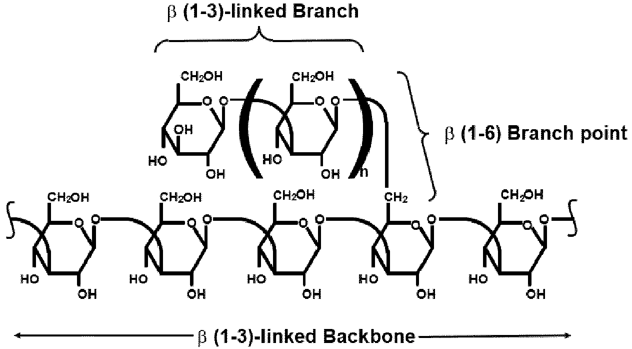

11. The effective amount of a *Saccharomyces cerevisiae*-derived (1→3)-β-D-glucan for use of any one of the preceding claims, wherein the WGP has a size of from about 1 to about 6 microns (or µm), or from about 2 to about 5 microns, or from about 3 to about 4 microns.

12. The effective amount of a *Saccharomyces cerevisiae*-derived (1→3)-β-D-glucan for use of any one of the preceding claims, provided as a food supplement comprising the *Saccharomyces cerevisiae*-derived (1→3)-β-D-glucan.

13. The effective amount of a *Saccharomyces cerevisiae*-derived (1→3)-β-D-glucan for use of any one of the preceding claims, wherein the food supplement comprises ≥ 75% beta 1,3/1,6 glucan on a dry weight basis.

14. The effective amount of a *Saccharomyces cerevisiae*-derived (1→3)-β-D-glucan for use of any one of the preceding claims, wherein the *Saccharomyces cerevisiae-*derived (1→3)-β-D-glucan is Wellmune^{®}.

15. An effective amount of a *Saccharomyces cerevisiae*-derived (1→3)-β-D-glucan for use in a method to induce trained innate immunity in a subject.
